(19) Europäisches Patentamt
European Patent Office
Office européen des brevets

(11) EP 1 396 490 A1

(12) **EUROPEAN PATENT APPLICATION**
published in accordance with Art. 158(3) EPC

(43) Date of publication:
**10.03.2004 Bulletin 2004/11**

(21) Application number: **02728131.0**

(22) Date of filing: **23.05.2002**

(51) Int Cl.[7]: **C07D 401/04**, C07D 401/14, C07D 405/14, C07D 409/14, C07D 413/14, C07D 417/14, C07D 471/04, C07D 487/04, C07D 491/113, A61K 31/4709, A61K 31/496, A61K 31/501, A61K 31/506, A61K 31/5355, A61K 31/5377, A61K 31/551, A61P 27/02, A61P 35/00, A61P 43/00

(86) International application number:
**PCT/JP2002/005014**

(87) International publication number:
**WO 2002/094809 (28.11.2002 Gazette 2002/48)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **24.05.2001 JP 2001155761**

(71) Applicant: **YAMANOUCHI PHARMACEUTICAL CO. LTD.**
**Tokyo 103-8411 (JP)**

(72) Inventors:
- **SAMIZU, K.,**
**Yamanouchi Pharmaceutical Co., Ltd.**
**Tsukuba-shi, Ibaraki 305-8585 (JP)**
- **HISAMICHI, H.,**
**Yamanouchi Pharmaceutical Co., Ltd.**
**Tsukuba-shi, Ibaraki 305-8585 (JP)**
- **MATSUHISA, A.,**
**Yamanouchi Pharmaceutical Co., Ltd.**
**Tsukuba-shi, Ibaraki 305-8585 (JP)**
- **KINOYAMA, I.,**
**Yamanouchi Pharmaceutical Co., Ltd.**
**Tsukuba-shi, Ibaraki 305-8585 (JP)**
- **HAYAKAWA, M.,**
**Yamanouchi Pharmaceutical Co., Ltd.**
**Tsukuba-shi, Ibaraki 305-8585 (JP)**
- **TANIGUCHI, N.,**
**Yamanouchi Pharmaceutical Co., Ltd.**
**Tsukuba-shi, Ibaraki 305-8585 (JP)**
- **IDEYAMA, Y.,**
**Yamanouchi Pharmaceutical Co., Ltd.**
**Tsukuba-shi, Ibaraki 305-8585 (JP)**
- **KUROMITSU, S.,**
**Yamanouchi Pharmaceutical Co., Ltd.**
**Tsukuba-shi, Ibaraki 305-8585 (JP)**
- **YAHIRO, K.,**
**Yamanouchi Pharmaceutical Co., Ltd.**
**Tsukuba-shi, Ibaraki 305-8585 (JP)**
- **OKADA, M.,**
**Yamanouchi Pharmaceutical Co., Ltd.**
**Tsukuba-shi, Ibaraki 305-8585 (JP)**

(74) Representative: **Bates, Philip Ian**
**Reddie & Grose**
**16 Theobalds Road**
**London WC1X 8PL (GB)**

(54) **3-QUINOLINE-2-(1H)-YLIDENEINDOLIN-2-ONE DERIVATIVES**

(57) There is provided medicaments, particularly a vascular endothelial growth factor (VEGF) inhibitor which is useful as a therapeutic drug for solid tumors, diabetic retinopathy and the like diseases in which angiogenesis is taking a role. That is, since a novel 3-quinolin-2(1H)-ylideneindolin-2-one derivative or a salt thereof has good VEGF inhibitory action, angiogenesis inhibitory action and anti-tumor action, it is useful as ideal VEGF inhibitor, angiogenesis inhibitor and anti-tumor agent.

EP 1 396 490 A1

Printed by Jouve, 75001 PARIS (FR)

## Description

Technical Field

This invention relates to medicaments, particularly a vascular endothelial growth factor (VEGF) inhibitor which is useful as a therapeutic drug for diseases in which angiogenesis is taking a role, such as cancers, diabetic retinopathy and the like.

Background of the Invention

It is known that several diseases accompany pathological angiogenesis closely related to their symptoms and causes. Typical disease among them is cancer, particularly a solid tumor, and it is necessary that a blood vessel newly formed from an already existing blood vessel elongates and reaches a tumor tissue, for the tumor tissue to grow to a diameter of exceeding 1 to 2 mm (*J. Natl. Cancer Inst.*, 82, 4 (1990))), and growth of the tumor tissue is explosively accelerated once the blood vessel reaches the tumor tissue. Also, a pathological angiogenesis accompanies in the retina in the case of diabetic retinopathy and frequently causes the loss of eyesight. In addition, a pathological angiogenesis is accompanied also by diseases such as rheumatoid arthritis, psoriasis, hemangioma, scleroderma, neovascular glaucoma and the like, and it is becoming one of the main symptoms (*N. Engl. J. Med.*, 320, 1211 (1989)). Accordingly, there is a possibility that a substance which inhibits angiogenesis can be applied to the treatment of solid tumors and the aforementioned diseases.

Vascular endothelial cells are cells which form innermost layer of a blood vessel. Angiogenesis is carried out by the proliferation of endothelial cells triggered by a growth factor or a physiologically active substance or by undergoing a stimulus such as a physical injury or the like. Though there are several growth factors that directly or indirectly stimulate growth of vascular endothelial cells, a vascular endothelial growth factor (VEGF) is known as a factor which is distinguished from other growth factors in terms that it acts upon vascular endothelial cells markedly specifically. That is, it has been reported that the VEGF receptors are vascular endothelium-selective, because they are expressed in very limited cells other than the vascular endothelial cells (*J. Clin. Invest.*, 89, 244 - 253 (1992)).

There are the following reports which suggest relationship between VEGF and cancers. Many cancer cells secrete VEGF *(Biochem. Biophys. Res. Commun.*, 194, 1234 (1993)). A cancer tissue and newly formed blood vessel in its periphery are strongly stained when a cancer tissue section is stained with an anti-VEGF antibody *(J. Exp. Med.*, 174, 1275 (1991), *Cancer Res.,* 53, 4727 (1993)). Growth of a transplanted cancer is inhibited in mice in which one of the VEGF receptors is genetically inactivated (*Nature*, 367, 576 (1994)). An anti-VEGF neutralizing antibody shows an anti-tumor activity in tumor bearing mice (*Nature*, 362, 841 (1993), *Biochem. Biophys. Res. Commun.*, 194, 1234 (1993)). Based on the above facts, it is considered that the VEGF secreted by cancer cells takes a central role in the tumor angiogenesis. In addition, since it is known that VEGF is also concerned in the acceleration of vascular permeability, this is considered to be one of the factors which cause malignant ascites and pleural effusion production.

Regarding the receptor of VEGF, two receptors Flt-1 and KDR/Flk-1 are known in the case of human (*FASEB J.*, 13, 9 - 22 (1999)). From a result of the disruption of these two genes, it has been shown that Flt-1 is concerned in the normal differentiation and morphogenesis of endothelial cells, and Flk-1 in the formation and growth of endothelial cells (*Nature*, 376, 66 - 70 (1995), *Nature,* 376, 62 - 66 (1995), *Nihon Yakurigaku Zasshi* (Japanese Journal of Pharmacology), 107, 119 - 131 (1996)). It is considered that VEGF binds to the Flk-1 receptor and accelerates growth of vascular endothelial cells via a tyrosine kinase-mediated signal transduction mechanism *(Proc. Natl. Acad. Sci. USA,* 88, 9026 - 9030 (1991)). It is shown also that VEGF has a direct *in vitro* angiogenesis inducing activity upon endothelial cells *(J. Cell. Physiol.,* 149, 50 - 59 (1991)).

Accordingly, it is expected that a VEGF inhibitor capable of inhibiting binding of VEGF with a VEGF receptor (particularly Flk-1) or inhibiting the VEGF signal transduction will inhibit angiogenesis and malignant ascites production and the like and therefore will be useful for the treatment of cancers, particularly solid tumors.

As the VEGF inhibitors, an anti-VEGF human monoclonal antibody (JP-A-9-316099) and some polypeptides (JP-A-9-255700, JP-A-9-154588) have been reported. Recently, low molecular weight compounds such as SU6668 *(Cancer Res.,* 60, 4152 - 4160 (2000)), PTK787/ZK222584 (*Cancer Res*., 60, 2178 - 2189 (2000)) shown below which can be orally administered and show VEGF inhibitory action have been reported.

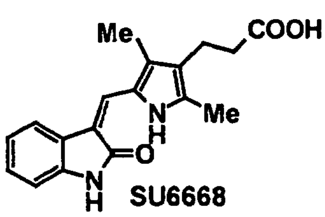

SU6668

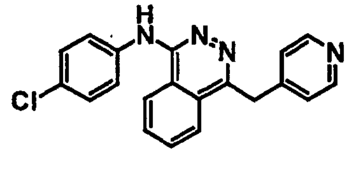

PTK787/ZK222584

In addition, quinazoline-substituted oxindol derivatives (WO 97/42187 and WO 99/10349) and pyrrolotriazine-substituted indolin-2-one derivatives (WO 00/71129) have been disclosed as useful compounds as a tyrosine kinase inhibitor and an angiogenesis inhibitor. However, there is no disclosure on their concrete pharmacological data.

Regarding the 3-quinolin-2(1H)-ylideneindolin-2-one derivative on the other hand, there are reports on the synthesis method of 3-quinolin-2(1H)-ylideneindolin-2-one as the compound (I') of the invention which will be described later wherein m and n are both 0 (to be referred to as compound A hereinafter) (*Ann. Chim. (Rome),* 57 (6), 688 - 97 (1967), *Chem. Pharm. Bull.*, 18 (9), 1822 - 30 (1970) and *Chem. Pharm. Bull.*, 19 (8), 1669 - 80 (1971)). However, there is no disclosure on its medicinal use.

Great concern is still directed toward the development of a vascular endothelial growth factor (VEGF) inhibitor useful as a therapeutic drug for diseases in which angiogenesis is concerned such as cancers, particularly solid tumors, diabetic retinopathy and the like, particularly a drug which can be orally administered.

Disclosure of the Invention

The present inventors have conducted extensive studies on a compound which inhibits angiogenesis based on the VEGF inhibitory action and found as a result that a 3-quinolin-2(1H)-ylideneindolin-2-one derivative in which the 2-position of the quinoline ring and the 3-position of the indolinone ring are directly bonded and the double bond is isomerized has good VEGF inhibitory action and is useful as an agent for the prevention or treatment of diseases which accompany angiogenesis wherein VEGF is concerned, thereby resulting in the accomplishment of the invention. As the compound in which the 2-position of the quinoline ring and the 3-position of the indolinone ring are directly bonded and the double bond is isomerized, only the aforementioned compound A is known, and there are no reports on its medicinal use. The fact that a compound having said nucleus has a good VEGF inhibitory action and is useful as a cancer treating drug is new information found by the present inventors.

That is, the present invention relates to a novel 3-quinolin-2(1H)-ylideneindolin-2-one derivative represented by the following general formula (I) or a salt thereof.

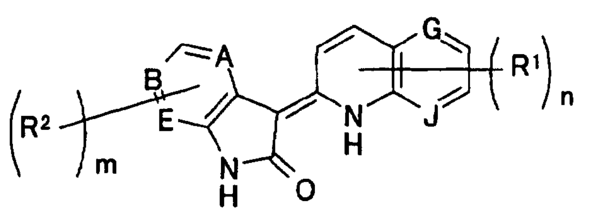

(I)

(Symbols in the formula have the following meanings;

A, B, E, G and J: the same or different from one another and each represents N atom or C atom,

$R^1$ and $R^2$: the same or different from each other and each represents a lower alkyl, a lower alkenyl, a lower alkynyl, $R^a$, X-($C_{1-8}$ alkylene which may be substituted by $OR^b$)-$R^a$, X-($C_{1-8}$ alkenylene)-$R^a$ or X-($C_{1-8}$ alkynylene)-$R^a$, with the proviso that each of $R^1$ and $R^2$ does not substitute to the ring nitrogen atom,

X: O, CO, COO, OCO, S, SO, $SO_2$, $NR^b$, $NR^bSO_2$, $SO_2NR^b$, $CONR^b$, $NR^bCO$, $NR^bCONR^c$, $NR^bCOO$, $OCONR^b$ or a bond,

$R^a$: a halogeno lower alkyl, a halogen, $NO_2$, CN, $OR^b$, O-(lower alkylene)-$NR^bR^c$, $COOR^b$, $COR^b$, $CONR^bR^c$, $NR^bR^c$, $NR^d$-(lower alkylene)-$NR^bR^c$, $NR^d$-(lower alkylene)-$OR^b$, N(lower alkylene-$NR^bR^c$)$_2$, $NR^cCOR^b$, $NR^d$-$CONR^bR^c$, $SR^b$, SO-lower alkyl, $SO_2$-lower alkyl, $SO_2$RIN, $SO_2$-(lower alkylene)-RIN, RIN, $SO_2NR^bR^c$, $NR^cSO_2R^b$,

$NR^cCOOR^b$, $OCO-NR^bR^c$, $OCO-R^b$, $NR^d$-(lower alkylene)-$COOR^b$, N(lower alkylene-$COOR^b$)$_2$, $CONR^b-OR^c$, CON-$R^d$-(lower alkylene)-$COOR^b$, CON(lower alkylene-$COOR^b$)$_2$, $CR^d=N-O-R^c$, $CR^d$=N-O-(lower alkylene)-$COOR^b$ or $CR^d$=N-O-(lower alkylene)-$NR^bR^c$,

$R^b$, $R^c$ and $R^d$: the same or different from one another and each represents H, a lower alkyl, a lower alkylene-RIN or RIN,

RIN: a saturated heterocyclic ring which may have one or more substituents, a cycloalkyl which may have one or more substituents, an aryl which may have one or more substituents or a heteroaryl which may have one or more substituents, and

n and m: the same or different from each other and each is 0 or an integer of from 1 to 4, with the proviso that at least one of n and m is an integer of from 1 to 4 when all of A, B, E, G and J are carbon atom, the same shall apply hereinafter.)

It further relates to novel pharmaceutical compositions which comprise a 3-quinolin-2(1H)-ylideneindolin-2-one derivative represented by the following general formula (I') or a salt thereof and a pharmaceutically acceptable carrier, particularly to a VEGF inhibitor, an angiogenesis inhibitor and an anti-tumor agent.

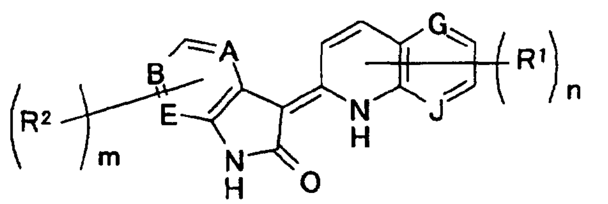
(I')

(Symbols in the formula have the following meanings;

A, B, E, G and J: the same or different from one another and each represents N atom or C atom,

$R^1$ and $R^2$: the same or different from each other and each represents a lower alkyl, a lower alkenyl, a lower alkynyl, $R^a$, X-($C_{1-8}$ alkylene which may be substituted by $OR^b$)-$R^a$, X-($C_{1-8}$ alkenylene)-$R^a$ or X-($C_{1-8}$ alkynylene)-$R^a$, with the proviso that each of $R^1$ and $R^2$ does not substitute to the ring nitrogen atom,

X: O, CO, COO, OCO, S, SO, $SO_2$, $NR^b$, $NR^bSO_2$, $SO_2NR^b$, $CONR^b$, $NR^bCO$, $NR^bCONR^c$, $NR^bCOO$, $OCONR^b$ or a bond,

$R^a$: a halogeno lower alkyl, a halogen, $NO_2$, CN, $OR^b$, O-(lower alkylene)-$NR^bR^c$, $COOR^b$, $COR^b$, $CONR^bR^c$, $NR^bR^c$, $NR^d$-(lower alkylene)-$NR^bR^c$, $NR^d$-(lower alkylene)-$OR^b$, N(lower alkylene-$NR^bR^c$)$_2$, $NR^cCOR^b$, $NR^d$-$CONR^bR^c$, $SR^b$, SO-lower alkyl, $SO_2$-lower alkyl, $SO_2$RIN, $SO_2$-(lower alkylene)-RIN, RIN, $SO_2NR^bR^c$, $NR^cSO_2R^b$, $NR^cCOOR^b$, $OCO-NR^bR^c$, $OCO-R^b$, $NR^d$-(lower alkylene)-$COOR^b$, N(lower alkylene-$COOR^b$)$_2$, $CONR^b-OR^c$, CON-$R^d$-(lower alkylene)-$COOR^b$, CON (lower alkylene-$COOR^b$)$_2$, $CR^d=N-O-R^c$, $CR^d$=N-O-(lower alkylene)-$COOR^b$ or $CR^d$=N-O-(lower alkylene) -$NR^bR^c$,

$R^b$, $R^c$ and $R^d$: the same or different from one another and each represents H, a lower alkyl, a lower alkylene-RIN or RIN,

RIN: a saturated heterocyclic ring which may have one or more substituents, a cycloalkyl which may have one or more substituents, an aryl which may have one or more substituents or a heteroaryl which may have one or more substituents, and

n and m: the same or different from each other and each is 0 or an integer of from 1 to 4, the same shall apply hereinafter.)

In this connection, the compound A known by the aforementioned references is included in the 3-quinolin-2(1H)-ylideneindolin-2-one derivative of the invention represented by the general formula (I').

The compounds of general formulae (I) and (I') are further described.

The term "lower" as used herein means a straight or branched hydrocarbon chain having from 1 to 6 carbon atoms. Accordingly, the "lower alkyl" is preferably an alkyl group having from 1 to 4 carbon atoms, particularly preferably a methyl, ethyl, propyl, isopropyl or isobutyl group. The "lower alkenyl" include preferably vinyl, allyl, 1-propenyl, isopropenyl, 1-butenyl, 2-butenyl and 3-butenyl groups. The "lower alkynyl" include preferably ethynyl, 1-propynyl, 2-propynyl, 1-butynyl, 2-butynyl, 3-butynyl and 1-methyl-2-propynyl groups. Also, preferred as the "lower alkylene" include methylene, ethylene, trimethylene and 2,2-dimethyltrimethylene groups. The "$C_{1-8}$ alkylene", "$C_{1-8}$ alkenylene" and "$C_{1-8}$ alkynylene" mean straight or branched chain alkylene, alkenylene and alkynylene groups having from 1 to 8 carbon atoms.

Regarding the "cycloalkyl", it is preferably a cycloalkyl group having from 3 to 8 carbon atoms and particularly preferably includes cyclopropyl, cyclopentyl and cyclohexyl. The "aryl" means an aromatic hydrocarbon ring group, and an aryl group having from 6 to 14 carbon atoms is preferable and it may be partially saturated. Preferred are phenyl

and naphthyl groups. The "heteroaryl" is a heteroaryl group having a five- or six-membered monocyclic ring containing from 1 to 4 hetero atoms selected from O, S and N, which may be condensed with benzene ring and/or partially saturated. Its preferred examples include furyl, thienyl, pyrrolyl, imidazolyl, pyrazolyl, furazanyl, thiazolyl, isothiazolyl, oxazolyl, isoxazole, oxadiazolyl, triazolyl, tetrazolyl, pyranyl, pyridyl, pyrimidinyl, pyridazinyl, pyrazinyl, indolyl, isoindolyl, benzimidazolyl, quinolyl, isquinolyl, dihydrooxazolyl, 5,6-dihydro-4H-oxazinyl, imidazolinyl, pyrrolinyl, pyrazolinyl, indolinyl, isoindilinyl and the like.

As the "halogen", F, Cl, Br and I atoms can be exemplified. The "halogeno lower alkyl" is the aforementioned lower alkyl group substituted by at least one of the aforementioned halogen atoms, and is preferably trifluoromethyl group.

The "saturated heterocyclic ring" is a 3- to 8-membered, preferably 5- to 7-membered, saturated heterocyclic ring group containing from 1 to 4 of N, O or S atom as a ring atom, which may have a cross linking or may form spiro-ring with another saturated heterocyclic ring or cycloalkyl (including 1,3-dioxolan and the like acetal compounds derived from oxo groups), and its preferred examples include aziridinyl, azetidinyl, pyrrolidinyl, piperazinyl, piperidyl, morpholinyl, thiomorpholinyl, pyrazolidinyl, imidazolidinyl, azepanyl, diazepanyl, quinuclidinyl, oxiranyl, tetrahydro-2H-pyranyl, tetrahydro-2H-thiopyranyl, dioxolanyl, oxetanyl, perhydrothiazinyl, tetrahydrothienyl and tetrahydrofuranyl groups, of which particularly preferred are piperazinyl, piperidyl, morpholinyl, tetrahydro-2H-pyranyl, tetrahydrofuranyl, dioxolanyl and oxetanyl groups.

In the aforementioned "heteroaryl" and "saturated heterocyclic ring", an optional C atom as a ring atom may be substituted by an oxo group, and a oxide or dioxide in which S or N atom is oxidized may be formed.

Regarding substituents of the "saturated heterocyclic ring which may have one or more substituents", "cycloalkyl which may have one or more substituents", "aryl which may have one or more substituents" and "heteroaryl which may have one or more substituents", they are preferably groups of the following G1 and G2 classes. More preferred are groups of the G2 class, further preferred are groups of a G3 class, and particularly preferred are groups of a G4 class. In this case, each of $R^e$, $R^f$ and $R^g$ represents H or a lower alkyl.

G1 class: a lower alkenyl, a lower alkynyl, a halogeno lower alkyl, $NO_2$, $NR^eCOR^f$, $NR^eCONR^fR^g$, $SR^e$, SO-lower alkyl, $SO_2$-lower alkyl and (aryl which may be substituted by one or more substituents selected from the group consisting of a lower alkyl, a halogen, $OR^e$, $NR^eR^f$, $COOR^e$, $COR^e$, $CONR^eR^f$ and an oxo group).

G2 class: (a lower alkyl which may be substituted by one or more substituents selected from the group consisting of $OR^e$, $NR^eR^f$, $COOR^e$, $COR^e$, $CONR^eR^f$, a cycloalkyl, a heteroaryl and (a saturated heterocyclic ring which may be substituted by one or more lower alkyl groups)), a halogen, $OR^e$, $NR^eR^f$, $COOR^e$, $COR^e$, $CONR^eR^f$, oxo, CN, (a saturated heterocyclic ring which may be substituted by one or more substituents selected from the group consisting of a lower alkyl, a halogen, $OR^e$, $NR^eR^f$, $COOR^e$, $COR^e$, $CONR^eR^f$ and an oxo group), a cycloalkyl and (a heteroaryl which may be substituted by one or more substituents selected from the group consisting of a lower alkyl, a halogen, $OR^e$, $NR^eR^f$, $COOR^e$, $COR^e$, $CONR^eR^f$ and an oxo group).

G3 group: {a lower alkyl which may be substituted by one or more substituents selected from the group consisting of $OR^e$, $NR^eR^f$, $COOR^e$, $COR^e$, $CONR^eR^f$, a heteroaryl and (a saturated heterocyclic ring which may be substituted by one or more lower alkyl groups)}, a halogen, $OR^e$, $NR^eR^f$, $COOR^e$, $COR^e$, $CONR^eR^f$, (a saturated heterocyclic ring which may be substituted by one or more lower alkyl groups), a cycloalkyl and a heteroaryl.

G4 class: a lower alkyl and a cycloalkyl.

According to the invention, two or more of the $R^1$ or $R^2$ are present when m or n is an integer of from 2 to 4, and respective members of $R^1$ or $R^2$ may be the same or different from one another. The $R^1$ may be substituted on any one of the 3- to 8-positions of the quinoline ring. A compound in which $R^1$ is substituted on the 5- or 6-position of the quinoline ring, and a compound in which $R^2$ is substituted on the 5- or 6-position of the indolinone ring are more preferable.

Among the compounds (I) and (I') of the invention, the following can be cited as preferred compounds.

(1) A compound in which A, B and E are (a) A and B are N atom and E is C atom, (b) one of A and E is N atom and the other is C atom, and B is C atom or (c) A, B and E are C atom; and G and J are both C atom or one of them is N atom and the other is C atom,

(2) a compound in which A, B, E, G and J are all C atom,

(3) a compound in which $R^1$ and $R^2$ may be the same or different from each other and each represents a lower alkyl, a lower alkenyl, a lower alkynyl, $R^a$, X-($C_{1-8}$ alkylene which may be substituted by OH)-$R^a$, X-($C_{1-8}$ alkenylene) -$R^a$ or X-($C_{1-8}$ alkynylene)-$R^a$; $R^a$ is a halogeno lower alkyl, a halogen, $NO_2$, CN, $OR^b$, O-(lower alkylene)-$NR^bR^c$, $COOR^b$, $COR^b$, $CONR^bR^c$, $NR^bR^c$, $NR^d$-(lower alkylene)-$NR^bR^c$, $NR^d$-(lower alkylene)-$OR^b$, N (lower alkylene-$NR^bR^c$)$_2$, $NR^cCOR^b$, $NR^dCONR^bR^c$, $SR^b$, SO-lower alkyl, $SO_2$-lower alkyl, RIN, $SO_2NR^bR^c$, $NR^cSO_2R^b$, $NR^cCOOR^b$ or OCO-$NR^bR^c$; and $R^b$, $R^c$ and $R^d$ may be the same or different from one another and each represents H, a lower alkyl or RIN,

(4) a compound in which X is O, CO, COO, S, $NR^b$, $CONR^b$, $NR^bCO$, $NR^bSO_2$, $NR^bCON^c$ or a bond,

(5) a compound in which m is 0, 1 or 2; $R^2$ is a lower alkyl, $R^{a2}$ or $X^2$-($C_{1-8}$ alkylene)-$R^{a2}$; $X^2$ is O, CO, COO, $NR^b$, $CONR^b$ or a bond; and $R^{a2}$ is a halogeno lower alkyl, a halogen, $NO_2$, CN, $OR^b$, $COOR^b$, $COR^b$, $CONR^bR^c$, $NR^bR^c$, $NR^cCOR^b$, $SO_2$-lower alkyl, RIN, $SO_2NR^bR^c$, OCO-$R^b$, $CONR^b$-$OR^c$, $CR^d$=N-$OR^c$ or $CR^d$=N-O-(lower alkylene) -$NR^bR^c$,

(6) a compound in which n is 0, 1 or 2; $R^1$ is a lower alkyl, $R^{a1}$ or $X^1$-($C_{1-8}$ alkylene which may be substituted with $OR^b$)-$R^{a1}$; $X^1$ is O, $CONR^b$, $NR^bCO$, $NR^bCONR^c$ or a bond; and $R^{a1}$ is a halogen, $NO_2$, CN, $OR^b$, $COOR^b$, $COR^b$, $CONR^bR^c$, $NR^bR^c$, $NR^d$-(lower alkylene)-$NR^bR^c$, $NR^d$-(lower alkylene)-$OR^b$, $NR^dCONR^bR^c$, RIN, OCO-$R^b$, $NR^d$-(lower alkylene)-$COOR^b$ or $CONR^b$-$OR^c$, and

(7) a compound in which n is 1 or 2 and $R^1$ is a halogen, a lower alkyl, CN, O-$C_{1-8}$ alkylene-(saturated heterocyclic ring which may be substituted by one or more substitutents selected from G2 class), $C_{1-8}$ alkylene-(saturated heterocyclic ring which may be substituted by one or more substitutents selected from G2 class), $C_{1-8}$ alkylene-$NR^e$-(lower alkylene)-(saturated heterocyclic ring which may be substituted by one or more substitutents selected from G2 class), CO-(saturated heterocyclic ring which may be substituted by one or more substitutents selected from G2 class), $C_{1-8}$ alkylene-(a substitutent selected from the group consisting of $COOR^e$, $NR^eR^f$, cycloalkyl and heteroaryl), or O-$C_{1-8}$ alkylene-(a substitutent selected from the group consisting of $COOR^e$, $NR^eR^f$, cycloalkyl and heteroaryl); and m is 0, 1 or 2 and $R^2$ is a halogen, a lower alkyl, $CO_2R^e$ or $CR^e$=N-$OR^f$,

(8) a compound in which n is 1 and $R^1$ is -$C_{1-8}$ alkylene-(saturated heterocyclic ring which may be substituted by one or more substitutents selected from G4 class), $C_{1-8}$ alkylene-(saturated heterocyclic ring which may be substituted by one or more substitutents selected from G4 class), $C_{1-8}$ alkylene-$NR^e$-(saturated heterocyclic ring which may be substituted by one or more substitutents selected from G4 class), $C_{1-8}$ alkylene-$NR^e$-(lower alkylene) -(saturated heterocyclic ring which may be substituted by one or more substitutents selected from G4 class), CO-(saturated heterocyclic ring which may be substituted by one or more substitutents selected from G4 class), $C_{1-8}$ alkylene-$COOR^e$, $C_{1-8}$ alkylene-$NR^eR^f$, or O-$C_{1-8}$ alkylene-$COOR^e$ and O-$C_{1-8}$ alkylene-$NR^eR^f$,

(9) a compound in which m is 1, $R^2$ is $CR^g$=N-O-(lower alkylene)-(heteroaryl which may be substituted by one or more substitutents selected from G2 class), and n is 0,

(10) a compound in which $R^2$ is $CR^g$=N-O-(lower alkylene)-(heteroaryl which may be substituted by one or more substitutents selected from G4 class), and

(11) compounds listed below and salts thereof, 3-[6-(2-morpholin-4-ylethoxy)quinolin-2(1H)-ylidene]indolin-2-one, 3-(6-{[(tetrahydro-2H-pyran-4-ylmethyl)amino]methyl}quinolin-2(1H)-ylidene)indolin-2-one, 3-{6-[(tetrahydro-2H-pyran-4-ylamino)methyl]quinolin-2(1H)-ylidene}indolin-2-one, 3-{6-[(4-methylpiperazin-1-yl)methyl]quinolin-2 (1H)-ylidene}indolin-2-one, 3-{6-[(4-ethylpiperazin-1-yl)methyl]quinolin-2(1H)-ylidene}indolin-2-one, 3-{6-[(4-cyclohexylpiperazin-1-yl)methyl]quinolin-2(1H)-ylidene}indolin-2-one, and 3-[6-(4-methylpiperazine-1-carbonyl)quinolin-2(1H)-ylidene]indolin-2-one.

The compounds (I) and (I') of the invention have theoretically possible two or more tautomers or stereoisomers in the conjugate system stretching from the 1-position nitrogen atom of the quinoline ring to the 1-position nitrogen atom of the indolinone ring, and separated forms or mixtures of these isomers are included in the invention.

Depending on the kinds of substituents, geometrical isomers and tautomers may be further present in the compounds of the invention, and separated forms or mixtures of these isomers are included in the invention. In addition, since the compounds of the invention have asymmetric carbon atoms in some cases, isomers based on these asymmetric carbon atoms can exist. Mixtures and separated forms of these optical isomers are included in the invention.

Also, the compounds of the invention form salts in some cases. Though not particularly limited so far as they are pharmaceutically acceptable salts, illustrative examples of the acid addition salt include acid addition salts with inorganic acids (e.g., hydrochloric acid, hydrobromic acid, hydroiodic acid, sulfuric acid, nitric acid, phosphoric acid and the like) and organic acids (e.g., formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid and the like), and examples of the salt with base include salts with inorganic bases containing metals (e.g., sodium, potassium, magnesium, calcium, aluminum and the like) or with organic bases (e.g., methylamine, ethylamine, ethanolamine, lysine, ornithine and the like), and ammonium salts. Still more, the invention also includes various hydrates, solvates and polymorphic substances of the compound (I) of the invention and salts thereof.

In addition, pharmacologically acceptable prodrugs are also included in the compounds of the invention. The pharmacologically acceptable prodrug is a compound having a group which is converted into $NH_2$, NH, OH, $CO_2H$ or the like of the invention by solvolysis or under a physiological condition. Examples of the group for forming a prodrug include those which are described in *Prog. Med.,* 5, 2157 - 2161 (1985) and "Iyakuhin no Kaihatsu (Development of Medicaments)" (Hirokawa Shoten, 1990) vol. 7, Bunshi Sekkei (Molecular Designing) 163 - 198. For example, OCO-(lower alkylene which may have one or more substituents)-COOR (R represents H or a lower alkyl, the same shall apply hereinafter), OCO-(lower alkenylene which may have one or more substituents)-COOR , OCO-(aryl which

may have one or more substituents), OCO-(lower alkylene)-O-(lower alkylene)-COOR, OCO-COR, OCO-(a lower alkyl which may have one or more substituents), $OSO_2$-(a lower alkylene which may have one or more substituents)-COOR, O-phthalidyl, 5-methyl-1,3-dioxolen-2-on-4-yl-methyloxy or the like is suitable as the group which is converted into OH; OCHR-O-CO-lower alkyl, OCHRO-CO-O-lower alkyl, 5-methyl-1,3-dioxolen-2-on-4-yl-methyloxy or the like is suitable as the group which is converted into $CO_2H$; and NHCO-$OCH_2$-OCO-lower alkyl, NCONH-lower alkyl, 2-tetrahydrofurfurylamino, 1-pyrrolidylmethylamino, an $NCH_2OCO$-lower alkyl, 5-methyl-1,3-dioxolen-2-on-4-yl-methyloxycarbonylamino or the like is suitable as the group which is converted into $NH_2$ or NH.

(Production methods)

The compound of the invention can be easily produced by those methods which are similar to the methods described in references, e.g., *Chem. Pharm. Bull.*, 18 (9), 1822 - 30 (1970), *J. Am. Chem. Soc.*, 122 (7), 1360 - 70 (2000) and the like, or methods known to those skilled in the art.

In this connection, depending on the kinds of functional groups, it is effective in some cases from the viewpoint of production techniques to replace said functional groups with appropriate protecting groups, namely those groups which can be converted into said functional groups, at the stage of the starting materials or intermediates. Thereafter, the desired compound can be obtained by removing the protecting groups as occasion demands. Examples of such functional groups include those groups which are described in "Protective Groups in Organic Synthesis" 3rd edition, edited by Greene and Wuts, and these may be optionally used in response to the reaction conditions.

Typical production methods are described in the following.

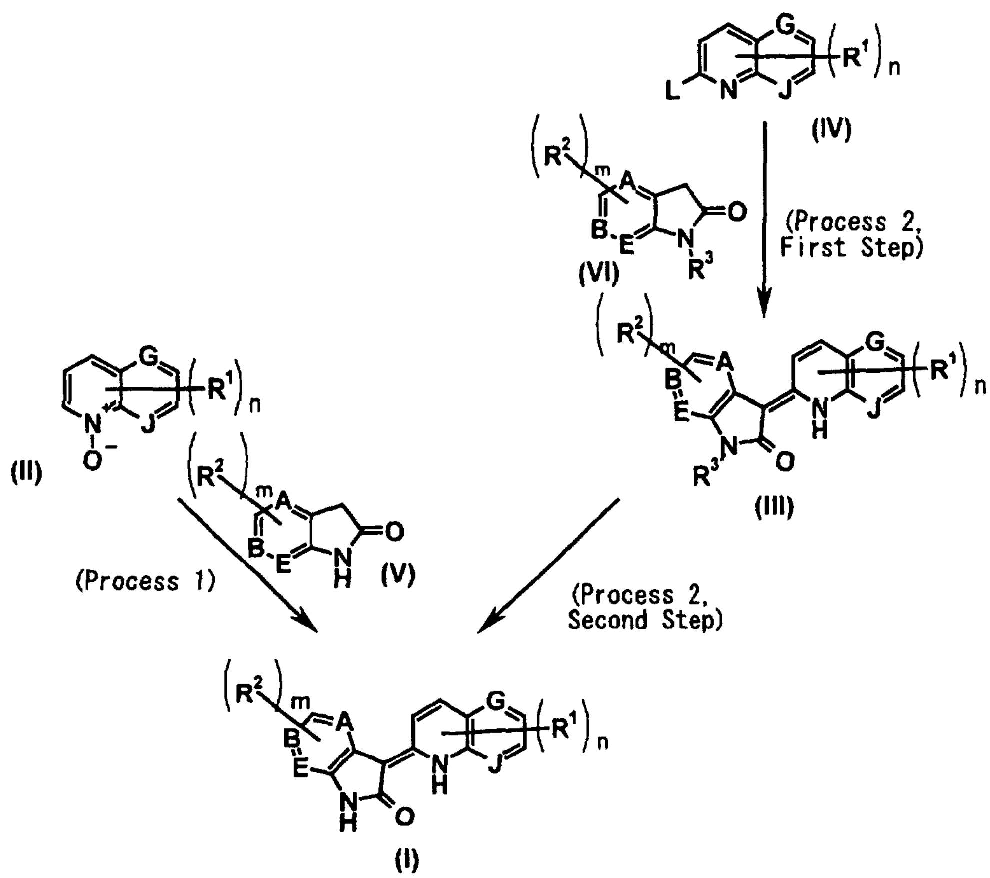


(In the formula, $R^3$ represents a protecting group such as diethoxymethyl, p-toluenesulfonyl, trimethylsilylethylsulfonyl or the like, and L represents a leaving group applicable to said reaction, such as a halogen, sulfonate or the like. The same shall apply hereinafter.)

First production method

The compound (I) of the invention can be produced by allowing a quinoline N-oxide compound (II) to react with an indolinone (V) in the usual way. The reaction can be carried out by optionally applying the method described, for example, in *Ann. Chim. (Rome)*, 57 (6), 188 - 97 (1967), *Khim. Geterotsikl. Soedin.,* 10, 1371 - 3 (1970), *Chem. Pharm. Bull.*, 18 (9), 1822 - 30 (1970) and *Chem. Pharm.* Bull., 19 (8), 1669 - 80 (1971), and it is advantageous to carry out the reaction in a solvent inert to the reaction (e.g., chloroform, acetonitrile or the like) at ordinary temperature or under heating, preferably at reflux temperature of the solvent, using reaction-corresponding amounts of the compounds (II) and (V) or one of them in excess amount, and using an appropriate acylation agent (benzoyl chloride, acetic anhydride or the like), sulfonylation agent (p-toluenesulfonyl chloride or the like), alkylation agent (methane iodide or the like) or silylation agent (chlorotrimethylsilane or the like) as the activation agent. When acetic anhydride is used, it is advantageous to use it as the solvent, it is desirable to carry out the reaction at ordinary temperature or under heating.

Second production method

In the first step, the compound (III) can be produced in accordance with the method described in *J. Am. Chem. Soc.,* 122 (7), 1360 - 70 (2000) or the like, by carrying out the reaction in a solvent inert to the reaction (e.g., toluene, tetrahydrofuran (THF) or the like) at ordinary temperature or under heating using reaction-corresponding amounts of the compounds (IV) and (VI) or one of them in excess amount, in the presence of a base (e.g., sodium tert-butoxide or the like), and by treating with a palladium complex (e.g., palladium acetate, palladium chloride, dibenzylideneacetone dipalladium or the like). The reaction will progress advantageously in some cases when a ligand of the palladium complex (e.g., BINAP, DPPF, Xantphos or the like) is added as occasion demands.

Next, the compound (I) of the invention can be produced in the second step, by deprotecting the compound (III) in the presence of an acid (e.g., hydrochloric acid or the like) in accordance with the method described in WO 97/42187 or the like, or in the presence of a reducing agent (e.g., tributyltin hydride or the like) in accordance with the method described in *Tetrahedron*, 56 (7), 979 - 988 (2000) or the like.

Third production method

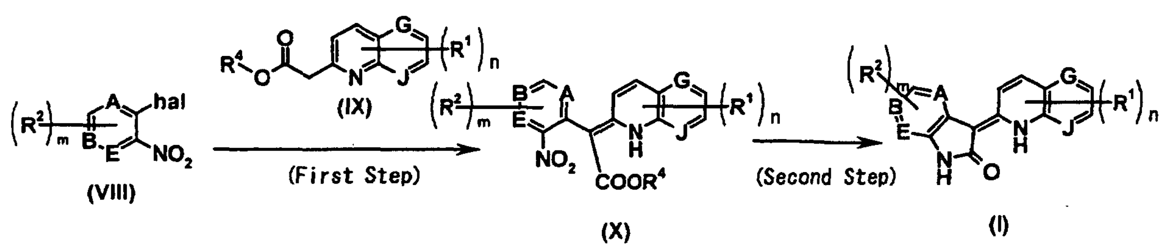


(In the formula, hal represents a halogen, and $R^4$ represents a lower alkyl. The same shall apply hereinafter.)

The first step can be easily carried out in accordance with known reaction conditions (e.g., *J. Med. Chem.*, 42, 5120 - 5130 (1999) or the like). The compound (X) can be produced by carrying out the reaction in a solvent inert to the reaction (e.g., N,N-dimethylformamide (DMF), dimethyl sulfoxide (DMSO), THF or the like) at ordinary temperature or under heating using reaction-corresponding amounts of the compounds (VIII) and (IX) or one of them in excess amount, in the presence of a base (e.g., sodium hydride, sodium tert-butoxide or the like) or an acid (e.g., acetic acid). Next, the compound (I) of the invention can be produced in the second step, by reducing nitro group of the compound (X) in accordance with a conventional reducing reaction such as the method described in *J. Med. Chem.*, 42, 5120 - 5130 (1999) or the like. The reaction will progress advantageously in some cases when heated or pressurized as occasion demands. It is possible to carry out conversion of a substituent in the compound (X) during this process by employing conditions of a conventional method. For example, when $R^2$ is a leaving group typified by a halogen or the like, it can be replaced by an amine derivative by an ipso substitution reaction, and when $R^1$ and $R^2$ are aldehyde, ketone and the like, they can be converted into oxime compounds and the like by condensation reaction and the like.

Other production methods

The compound of the invention can be produced by various known substituent modification reactions, in

addition to the aforementioned production methods. For example, it can be easily produced with reference to the conditions described in references or cited references therein such as COMPREHENSIVE ORGANIC SYNTHESIS, edited by B.M. Trost (Pergamon Press) (1991), COMPREHENSIVE ORGANIC TRANSFORMATIONS, edited by R.C. Larock (VCH Publishers) (1989), ADVANCED ORGANIC CHEMISTRY, edited by J. March (John WILEY & SON) (1992), "Jikken Kagaku Koza (Experimental Chemistry Course)" 4th edition, edited by The Chemical Society of Japan (Maruzen) or the like. Main production methods are described in the following.

A compound having an aminoalkyl group-containing substituent can be easily produced (1) from a compound having a halogen-substituted alkyl group or an epoxide by a conventional amination reaction, (2) from a compound having an aldehyde or ketone by a conventional reductive amination reaction (e.g., *Tetrahedron Lett.*, 31, 5595 - 5598 (1990) or the like can be used as a reference) or (3) from a compound having a protected aminoalkyl group by a deprotection reaction (e.g., treatment with hydrochloric acid, trifluoroacetate (TFA) or the like in the case of tert-butoxycarbonyl group (Boc) or treatment with hydrazine or methylamine in the case of phthalimido group).

In case that reaction of the reductive amination reaction hardly progresses by the use of a ketone or a secondary amine or a combination of a ketone and a secondary amine, it is desirable to produce the compound by a method similar to the method described, for example, in *J. Org. Chem.*, 55 (8), 2552 (1990).

A compound having an ether bond-containing substituent can be produced from a compound having phenol or hydroxyl group by conventional O-alkylation reaction or the Mitsunobu reaction described, for example, in *Tetrahedron Lett.*, 40 (4), 671 - 674 (1999), *Chem. Lett.*, (2), 97 - 98 (1996) or *Helv. Chim. Acta*, 81 (5), 865 - 880 (1998). A compound having an amino group-containing substituent can be produced from a compound having nitro group via a conventional reduction reaction.

A compound having hydroxyl group can be produced (1) from a compound having an ester group or the like by a conventional hydrolysis reaction or (2) from a compound having benzyl group or the like by a conventional hydrogenolysis reaction. A compound having carboxyl group can be produced from a compound having an ester group by a conventional hydrolysis reaction, particularly by a hydrogenolysis in the case of an ester comprising benzyl alcohol or the like. A compound having amido bond can be produced by a conventional amidation reaction which uses an amino group-containing compound of the invention and an acid chloride, a mixed acid anhydride, carbodiimide or the like, or a conventional amidation reaction which uses a carboxyl group-containing compound of the invention and an amine.

A compound having a hydroxamic acid ester can be produced from a carboxyl group-containing compound of the invention by a conventional amidation reaction which uses hydroxylamines. A compound having an *O*-substituted oxime-containing substituent can be produced from an aldehyde- or ketone-containing compound of the invention by a conventional dehydration condensation reaction or the like which uses *O*-substituted hydroxylamines. A compound having urea bond-containing substituent can be produced from a compound having amino group by a conventional urea introducing reaction or the like which uses an isocyanate or mediates a phenylcarbamate derivative.

An N-oxide compound can be produced by a known oxidation reaction, namely by the reaction with an oxidation agent (e.g., m-chloroperbenzoic acid, hydrogen peroxide or the like) in a reaction-inert solvent (e.g., chloroform, dichloromethane or the like). It is possible to convert a sulfide into a sulfoxide or a sulfone under the same oxidation condition. When it is planned to obtain a desired compound from a precursor compound having an N-hydroxyamido bond by carrying out a de-hydroxylation reaction, it can be easily carried out by mediating a conventional reducing condition (e.g., a reaction with metallic iron in acetic acid, a hydrogenolysis reaction or the like). Introduction of an aromatic hetero ring can be easily carried out by a method in which a substituent having a precursor is introduced and then converted into a hetero ring by a conventional condensation reaction.

(Synthesis of Starting Compounds)

Some of the starting compounds of the compound of the invention are novel compounds, and these compounds can be easily synthesized in the same manner as the known starting compounds or using certain methods known to those skilled in the art. Typical synthesis methods are shown below.

Synthesis method 1 (alkylation) References: *J. Med. Chem.*, 40, 1252 - 1257 (1997) and the like

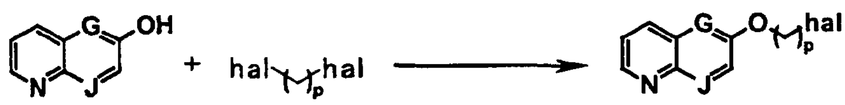

(VIIa) (VIIb)

(In the formula, p is an integer of 1 to 8. The same shall apply hereinafter.)

Synthesis method 2 (oxidation) References: *Synthesis*, 87 - 90 (1997) and the like

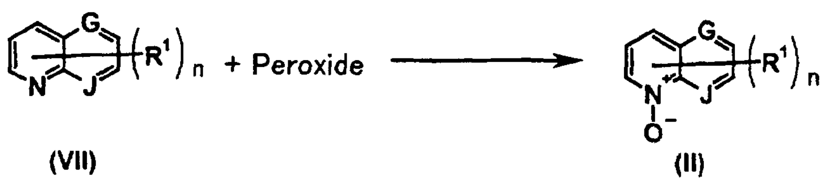


(VII) (II)

Synthesis method 3 References: *J. Heterocyclic Chem.*, 15, 1425 - 1430 (1978) and the like

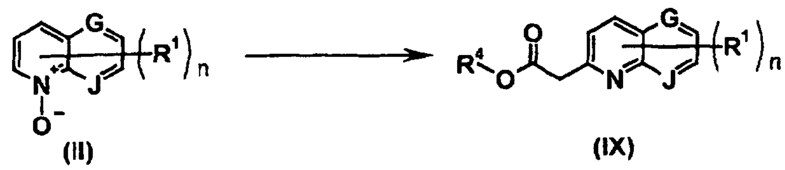

(II) (IX)

The quinolineacetic acid derivative (IX) can be produced in the usual way by treating the compound (II) with ethyl acetoacetate, diethyl malonate or the like in the presence of an appropriate acylation agent, sulfonylation agent, alkylation agent or silylation agent.

Synthesis method 4 (Sonogashira reaction) References: *Synthesis*, 364 - 365 (1981) and the like

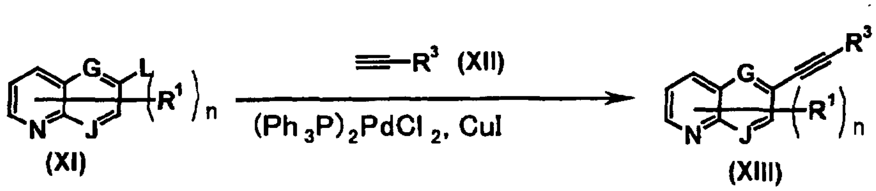


(XI) (XIII)

(In the formula, $R^3$ represents a substituted alkyl or O-substituted alkyl.)

Synthesis method 5 (halogenation) References: *J. Am. Chem. Soc.,* 77, 1054 - 1055 (1955), *Tetrahedron*, 54, 13655 - 13680 (1998) and the like

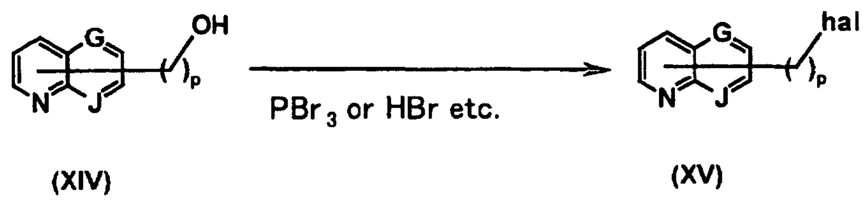


Synthesis methods for other starting compounds

A compound having a substituent group on the quinoline ring can also be produced, for example, by employing the methods described in *Heterocycles,* 54, 105 - 108 (2001) and *J. Med. Chem*., 26, 580 - 585 (1983), or a method in which a 4-chloroquinoline derivative is produced by applying *Org. Synth. Col.,* Vol. 3, 272 (1955), *Syn. Commun*., 15, 125 (1995) or the like and then the chloro group is removed by a conventional method via a reducing condition and the like.

Regarding synthesis of the indolinone ring, it can be easily produced by employing the conditions described in *Synthesis*, 51 - 53 (1993), *Eur. J. Med. Chem*., 15, 330 - 332 (1980) or the like.

Introduction of a substituent group onto the indolinone ring can be carried out by applying the Suzuki-Miyaura reaction of its halogen derivative or the Friedel-Crafts reaction, and a conversion reaction or the like into the aromatic hetero ring via a condensation reaction which uses the introduced $\alpha$-halo ketone group. For example, it is possible to employ the methods described in *J. Med. Chem*., 42, 5120 - 5130 (1999), *Synthesis,* 873 - 874 (1989), *J. Org. Chem*., 17, 1252 - 1255 (1952) and the like. Also, it is possible to remove the introduced primary amine on the aromatic hetero ring, for example by applying the method described in *J. Med. Chem*., 39, 834 - 841 (1996).

In addition, as occasion demands, the starting compounds of interest can be produced by subjecting to amination, imination, acylation, alkylation, amidation, sulfonamidation, esterification, urea introducing reaction, halogenation, nitration, oxidation, reduction, protection, deprotection and the like various known substituent group modification reactions. These reactions can be carried out with reference to the conditions described in the aforementioned references such as "Jikken Kagaku Koza" 4th edition, edited by The Chemical Society of Japan (Maruzen) or the like.

Isolation and purification of the compound of the invention produced in this manner are carried out by employing extraction, concentration, crystallization, filtration, recrystallization, various chromatography techniques and the like general chemical operations.

Each of the isomers can be isolated in the usual way by making use of a difference in the physicochemical property between isomers. For example, a racemic compound can be separated to optically pure isomer by a general optical resolution method [e.g., a method in which the compound is introduced into diastereomer salts with a general optically active acid (tartaric acid or the like) and then subjected to optical resolution]. Also, a mixture of diastereomers can be separated for example by fractional crystallization, a chromatography and the like. In addition, an optically active compound can also be produced by the use of an appropriate optically active material.

Industrial Applicability

Since the drug of the invention has a VEGF inhibitory action, it is useful in the treatment and improvement of diseases and morbid states in which VEGF is taking a role. Particularly, as an inhibitor of angiogenesis caused by VEGF, it is useful for the growth inhibition of cancers, particularly solid tumors, hemangioma and the like tumors, for the prevention and treatment of rheumatoid arthritis, psoriasis, scleroderma and the like diseases, and for the prevention and treatment of diabetic retinopathy and the like retinal diseases and neovascular glaucoma and the like eye diseases.

As is shown in the following test examples, the compound of the invention showed good inhibitory activity upon the VEGF-stimulated growth of vascular endothelial cells. Also, the compound of the invention inhibited VEGF-dependent *in vitro* angiogenesis. Accordingly, it was revealed that the compound of the invention inhibits growth and angiogenesis of vascular endothelial cells caused by VEGF.

Since it has been confirmed that the compound of the invention inhibits cancer growth having a significance against control when it is orally administered in an anti-tumor test using COLO 205 (human colon tumor)-bearing nude

mice, it was suggested that it inhibits growth of cancer via its action to inhibit VEGF-induced angiogenesis. Accordingly, it is useful as an angiogenesis inhibitor and an anti-tumor agent, which can be orally administered.

In addition, since the compound of the invention inhibits acceleration of vascular permeability caused by VEGF, it is also useful as an agent for improving malignant ascites and pleural effusion production.

Test Example 1 Test on the inhibition of VEGF-stimulated HUVEC growth

Test method: (Cell culture) Human umbilical vein endothelial cells (HUVEC) were cultured using EGM-2 complete medium (Clonetics) which had been supplemented with additives (2% FBS, 0.4% FGF (fibroblast growth factor), 0.1% VEGF, 0.1% IGF-I (insulin like growth factor-I), 0.1% EGF (epidermal growth factor), 0.1% ascorbic acid, 0.1% GA-1000, 0.1% heparin and 0.04% hydrocortisone).

(Evaluation of compounds) HUVEC (10,000 cells/well) were inoculated into EGM-2 complete medium in a gelatin-coated 96 well plate (mfd. by IWAKI) and cultured overnight. After washing with a physiological phosphate buffer, the medium was exchanged with a low serum medium (Medium 199/0.1% FBS) and the culturing was continued for 24 hours. Each of the compounds to be evaluated was prepared as a 10 mM DMSO solution and diluted with the low serum medium. This was added to each well to a final concentration of from 0.001 to 10 μM. After 2 hours of the compound treatment, human recombinant VEGF (R & D Systems) was added to a final concentration of 10 ng/ml. After 18 hours, [$^3$H]thymidine (Amersham Pharmacia) was added in 5 μCi/well portions. After 4 hours, the reaction was terminated by adding 0.2% SDS in 50 μl/well portions, and the product was recovered on GF/C Unifilter (Packard). After adding 25 μl of Microscinti 20 (Packard), the radioactivity incorporated into the DNA trapped on the filter was measured using TopCount (Packard). The $IC_{50}$ of the inhibitory activity of each compound was calculated as the 50% inhibition concentration ($IC_{50}$ value) of each compound to be tested, by defining the incorporated amount of [$^3$H]thymidine at the time of VEGF addition as 100%, and its incorporated amount at the time of no addition of VEGF as 0%.

Results: The results are shown in the following table. It was confirmed by this test that the compounds of the invention have good VEGF inhibitory action.

Table 1

| Ex. No. | $IC_{50}$ (μM) | Ex. No. | $IC_{50}$ (μM) | Ex. No. | $IC_{50}$ (μM) | Ex. No. | $IC_{50}$ (μM) |
|---|---|---|---|---|---|---|---|
| **Compound A** | 0.14 | **47** | 0.17 | **111** | 0.30 | **239** | 0.31 |
| **1** | 0.21 | **48** | 0.54 | **112** | 0.10 | **242** | 0.045 |
| **4** | 0.19 | **53** | 0.16 | **144** | 0.093 | **259** | 0.071 |
| **8** | 0.17 | **54** | 0.029 | **145** | 0.031 | **265** | 0.18 |
| **9** | 0.20 | **56** | 0.11 | **146** | 0.076 | **283** | 0.084 |
| **10** | 0.99 | **57** | 0.72 | **148** | 0.074 | **284** | 0.36 |
| **14** | 0.30 | **58** | 0.034 | **149** | 0.073 | **285** | 0.070 |
| **15** | 0.32 | **59** | 0.087 | **159** | 0.038 | **286** | 0.75 |
| **16** | 0.11 | **66** | 0.35 | **160** | 0.046 | **288** | 0.038 |
| **17** | 0.012 | **77** | 0.16 | **161** | 0.10 | **293** | 0.048 |
| **21** | 0.22 | **79** | 0.10 | **163** | 0.043 | **294** | 0.012 |
| **25** | 0.38 | **80** | 0.10 | **175** | 0.098 | **295** | 0.033 |
| **32** | 0.013 | **81** | 0.068 | **177** | 0.11 | **296** | 0.047 |
| **36** | 0.16 | **82** | 0.042 | **187** | 0.14 | **297** | 0.031 |
| **37** | 0.31 | **83** | 0.11 | **191** | 0.070 | **298** | 0.050 |
| **38** | 0.038 | **85** | 0.11 | **193** | 0.87 | **299** | 0.027 |
| **39** | 0.032 | **86** | 0.10 | **201** | 0.29 | **301** | 0.0076 |
| **40** | 0.0062 | **87** | 0.10 | **203** | 0.0048 | **310** | 0.31 |
| **41** | 0.12 | **96** | 0.15 | **204** | 0.022 | **311** | 0.24 |

Table 1 (continued)

| Ex. No. | $IC_{50}$ (μM) | Ex. No. | $IC_{50}$ (μM) | Ex. No. | $IC_{50}$ (μM) | Ex. No. | $IC_{50}$ (μM) |
|---|---|---|---|---|---|---|---|
| **42** | 0.11 | **98** | 0.15 | **209** | 0.018 | **312** | 0.048 |
| **44** | 0.28 | **99** | 0.13 | **213** | 0.00097 | **341** | 0.020 |
| **45** | 0.036 | **102** | 0.41 | **217** | 0.011 | **354** | 0.14 |
| **46** | 0.12 | **105** | 0.22 | **222** | 0.0068 | **361** | 0.024 |

Test Example 2 *In vitro* angiogenesis inhibition test

Test method: An angiogenesis assay kit (mfd. by KURABO) was used. A medium prepared by adding 10 ng/ml of VEGF (R & D Systems) to the special medium attached to the kit was used as the culture medium. Each test drug was prepared as a 10 mM DMSO solution and added to a final concentration of from 0.003 to 1 μM by diluting with the special medium. Amount of angiogenesis under a condition of not adding the test drug was used as the positive control, and a condition under which 25 μg/ml of an anti-VEGF antibody (Sigma) was added was used as the negative control. On the 4th, 7th and 10th days after commencement of the culturing, the test drug was prepared as described in the above and exchanged with the medium of each well. On the 13th day, fixation of the cell layer was carried out in accordance with the method attached to the kit. That is, fixation of cell layer was carried out by adding ice-cooled 70% ethanol after washing with a physiological phosphate buffer and then allowing to stand at room temperature for 30 minutes. Next, the ethanol solution was removed by sucking, followed by washing with a blocking solution (physiological phosphate buffer containing 1% BSA).

A endotherial vessel staining kit CD31 for staining (mfd. by KURABO) was used for the staining of the thus formed hollow organ. That is, the mouse anti-human CD31 antibody attached to the kit was diluted 4,000 times with the blocking solution and added to each well to carry out the incubation at 37°C for 60 minutes. Subsequently, after washing three times with the blocking solution, a goat anti-mouse IgG alkaline phosphatase conjugate solution diluted 500 times with the blocking solution was added to each well and incubated at 37°C for 60 minutes. After the incubation, each well was washed three times with distilled water. Next, a BCIP/NBT solution prepared by dissolving in distilled water was added to each well and incubated at room temperature for 5 to 10 minutes. After the incubation, this was washed three times with distilled water and then spontaneously dried. Under a microscope, the stained endotherial vessel image was photographed at 4 positions around the center of each well and preserved by a TIFF mode. An imaging software (ScnImage) shown in the angiogenesis kit was used for the determination of the endotherial vessel forming amount. By importing the file preserved in the imaging software, the number of pixel obtained by threshold and measure commands was recorded. Average of the number of pixel at the 4 positions obtained from 1 well was used as the endotherial vessel forming amount in said well.

The $IC_{50}$ value of each test drug was calculated as the 50% inhibiting concentration of the test drug, by defining the number of pixel in a positive control well under a test drug-non-added condition as 100%, and the number of pixel in a negative control well under an anti-VEGF antibody-added condition as 0%.

Results: The compounds of the invention showed excellent activity in this test; for example, $IC_{50}$ value of the compound A was 0.069 μM.

Test Example 3 *In vivo* anti-tumor test using human colon tumor bearing nude mice

Test method: A total of 4 x $10^6$ cells of a human colon tumor COLO 205 were administered under the dorsal side skin of each female Balb/c nude mouse. When the tumor volume reached 50 to 100 $mm^3$, each of the test compounds was orally administered once a day for 14 days. In addition, 0.5% methyl cellulose aqueous solution was orally administered to the control group. Calipers were used for the measurement of tumor diameter, and it was measured on the next day of the final administration. In this case, the tumor volume was calculated using the following calculation formula.

$$\text{Tumor volume} = (\text{width}^2 \times \text{length})/2$$

Results: It was confirmed by this test that the compounds of Examples 4, 41, 44, 54, 96, 99, 111, 113, 115, 132, 134, 148, 310 and 311 of the invention inhibit the tumor growth with a significance for the control, by their oral administration at a dose of 10 or 30 mg/kg/day.

The pharmaceutical composition which contains one or two or more of the compounds represented by the

general formula (I) or salts thereof as the active ingredient is prepared by a generally used method using pharmaceutical carriers, fillers and the like generally used in this field. It may be administered either by oral administration in the form of tablets, pills, capsules, granules, powders, solutions inhalations and the like, or by parenteral administration in the form of intravenous, intramuscular and the like injections, suppositories, ophthalmic solutions, ophthalmic ointments, solutions for percutaneous absorption, ointments, adhesives for percutaneous absorption, transmucosal solutions, transmucosal adhesive preparations and the like.

The solid composition for use in the oral administration according to the present invention is used in the form of tablets, powders, granules and the like. In such a solid composition, one or more active substances are mixed with at least one inert diluent such as lactose, mannitol, glucose, hydroxypropylcellulose, microcrystalline cellulose, starch, polyvinyl pyrrolidone, aluminum magnesium silicate or the like. In accordance with the usual way, the composition may contain other additives than the inert diluent, such as a lubricant (e.g., magnesium stearate or the like), a disintegrating agent (e.g., calcium cellulose glycolate or the like), a stabilizing agent and a solubilization assisting agent. If necessary, tablets or pills may be coated with a sugar coat or a film of a gastric or enteric substance such as sucrose, gelatin, hydroxypropylcellulose, hydroxypropylmethylcellulose phthalate or the like.

The liquid composition for oral administration includes pharmaceutically acceptable emulsions, solutions, suspensions, syrups, elixirs and the like and contains a generally used inert diluent such as purified water or ethanol. In addition to the inert diluent, this composition may also contain a moistening agent, a suspending agent and the like auxiliary agents, as well as sweeteners, flavors, aromatics and antiseptics.

The injections for parenteral administration includes aseptic aqueous or non-aqueous solutions, suspensions and emulsions. Examples of the diluent for use in the aqueous solutions and suspensions include distilled water for injection and physiological saline. Examples of the diluent for use in the non-aqueous solutions and suspensions include propylene glycol, polyethylene glycol, a plant oil (olive oil or the like), an alcohol (ethanol or the like), polysorbate 80 (trade name) and the like. Such a composition may further contain auxiliary agents such as an antiseptic, a moistening agent, an emulsifying agent, a dispersing agent, a stabilizing agent and a solubilization assisting agent. These compositions are sterilized by filtration through a bacteria retaining filter, blending of a germicide or irradiation. Alternatively, these may be used by producing sterile solid compositions and dissolving them in sterile water or a sterile solvent for injection prior to their use.

In general, a daily dose of approximately from 0.001 to 50 mg/kg, preferably from 0.01 to 10 mg/kg, per body weight in the case of oral administration, and a daily dose of approximately from 0.0001 to 5 mg/kg per body weight in the case of intravenous administration, are respectively suitable, and the daily dose is divided into 1 to several doses per day. The dose is optionally decided in response to each case by taking symptoms, age, sex and the like into consideration.

Best Mode for Carrying Out the Invention

The following describes the invention further in detail based on examples. Production methods of starting compounds to be used in Examples are shown in reference examples. In this connection, the compounds of the invention are not limited to the compounds described in the following Examples.

Also, abbreviations of physicochemical properties described in the reference examples, Examples and subsequent tables indicate,
F+: FAB-MS $(M + H)^+$; F-: FAB-MS $(M - H)^-$; F: FAB-MS $(M)^+$; E+: ESI-MS $(M + H)^+$; E: ESI-MS $(M)^+$; N1: characteristic peak δ ppm of $^1$H-NMR (DMSO-$d_6$, TMS internal standard) ; and N2: characteristic peak δ ppm of $^1$H-NMR ($CDCl_3$, TMS internal standard).

Reference Example A1: A DMF solution of ethyl quinolin-2-ylacetate was mixed with 60% NaH and stirred, and then N,N-diethyl-4-fluoro-3-nitrobenzamide was added thereto and stirred. By purifying the thus formed substance from the reaction solution, ethyl {4-[(diethylamino)carbonyl]-2-nitrophenyl} (quinolin-2(1H)-ylidene)acetate was obtained as a brown foam. F+: 435.

Reference Example A2: 2,4-Dichloro-5-nitropyrimidine was added to an acetic acid solution of ethyl quinolin-2-ylacetate and stirred at 50°C. After spontaneous cooling, the thus formed precipitate was collected by filtration to obtain ethyl (2-chloro-5-nitropyrimidin-4-yl)(quinolin-2(1H)-ylidene)acetate as a red solid. F+: 373.

Reference Example A3: Morpholine was added to a pyridine solution of ethyl (5-fluoro-2-nitrophenyl)(quinolin-2(1H)-ylidene)acetate and stirred at 100°C and then the mixture was purified to obtain ethyl (5-morpholin-4-yl-2-nitrophenyl)(quinolin-2(1H)-ylidene) acetate as a red solid. F+: 422.

Reference Example B1: Under ice-cooling, oxalyl chloride and a catalytic amount of DMF were added to a dichloromethane solution of 4-fluoro-3-nitrobenzoic acid and stirred. After evaporation of the solvent, the resulting residue was dissolved in THF and, under ice-cooling, added dropwise to a THF solution of O-(cyclopropylmethyl) hydroxylamine hydrochloride and triethylamine (TEA). After stirring the reaction solution, the thus formed substance was purified to obtain N-(cyclopropylmethoxy)-4-fluoro-3-nitrobenzamide as a yellow solid. F+: 255.

Reference Example B2: Ethyl chloroformate and TEA were added to a THF solution of 2-oxoindoline-5-carboxylic acid and stirred. The reaction solution was mixed with N,N-diethylethylenediamine, stirred and then purified to obtain N-[(2-diethylamino)ethyl]-2-oxoindoline-5-carboxamide as a brown solid. F+: 276.

Reference Example C: An acetonitrile solution of 6-(2-bromoethoxy)quinoline N-oxide was mixed with morpholine, stirred at 100°C and then purified to obtain 6-[(2-morpholin-4-yl)ethoxy]quinoline N-oxide as a light brown solid. F+: 275.

Reference Example D: Methoxylamine hydrochloride was added to a THF solution of 4-bromo-2-methyl-5-nitrobenzaldehyde and stirred at 50°C for 8 hours. By purifying the thus formed substance from the reaction solution, 4-bromo-2-methyl-5-nitrobenzaldehyde O-methyloxime was obtained as a colorless oil. F-: 272, 274.

Reference Example E1: A DMF solution of 6-hydroxyquinoline was mixed with 60% NaH, and stirred at 50°C. After spontaneous cooling, the reaction solution was mixed with 1-bromo-2-methoxyethane, stirred and then purified to obtain 6-(2-methoxyethoxy)quinoline as a yellow oil. F+: 220.

Reference Example E2: A DMSO solution of 7-hydroxyindolin-2-one was mixed with N-(2-chloroethyl)-N,N-diethylamine hydrochloride and potassium carbonate, stirred at room temperature for 30 minutes and then stirred at 50°C for 30 minutes, and the thus formed substance was purified to obtain 7-[2-(diethylamino)ethoxy]indolin-2-one as a yellow oil. F+: 249.

Reference Example E3: A THF solution of 6-hydroxyquinoline, 2-(1H-1,2,3-triazol-1-yl)ethanol and triphenylphosphine was mixed with diethyl azodicarboxylate, stirred and then purified to obtain 6-[2-(1H-1,2,3-triazol-1-yl) ethoxy]quinaline as a yellow solid. F+: 241.

Reference Example E4: 6-Hydroxyquinoline was suspended in 2 M sodium hydroxide aqueous solution and mixed with tetrabutylammonium hydrogensulfate and 1,2-dibromoethane, and the mixture was stirred at 60°C and then purified to obtain 6-(2-bromoethoxy)quinoline as a brown oil. F+: 252, 254.

Reference Example E5: A DMF solution of 2-[(6-hydroxyquinolin-5-yl)methyl]isoindoline-1,3-dione was mixed with 1-bromo-2-methoxyethane and cesium carbonate and stirred at 70°C. The reaction solution was mixed with saturated sodium bicarbonate aqueous solution, stirred under reflux and then spontaneously cooled to obtain 2-{[6-(2-methoxyethoxy)quinolin-5-yl]methyl}isoindoline-1,3-dione as a brown solid. F+: 363.

Reference Example F: Piperidin-2-one was added to a DMF solution of 60% NaH and stirred at 50°C. After spontaneous cooling, 6-(2-bromoethoxy)quinoline was added to the reaction solution and stirred, and then the product was purified to obtain 1-[2-(quinolin-6-yloxy)ethyl]piperidin-2-one as a yellow oil. F+: 271.

Reference Example G: Ethyl 3-oxobutyrate was added to an acetic anhydride solution of 6-(2-bromoethoxy) quinoline 1-oxide and stirred at 60°C. The reaction solution was alkalized and then extracted with ethyl acetate. The residue after evaporation of the solvent mixed with 4 M hydrochloric acid and stirred, and then the alkalized reaction solution was extracted with ethyl acetate. By purifying the thus formed substance, ethyl [6-(2-bromoethoxy)quinolin-2-yl]acetate was obtained. F+: 338, 340.

Reference Example H1: An ethyl acetate solution of 6-(2-bromoethoxy)quinoline was mixed with 70% m-chloroperbenzoic acid and stirred. The thus formed precipitate was collected by filtration to obtain 6-(2-bromoethoxy) quinoline N-oxide as a light yellow solid. F+: 268, 270.

Reference Example H2: A carbon tetrachloride solution of 2-(4-fluoro-3-nitrophenyl)-4,5-dihydro-1,3-oxazole was mixed with N-bromosuccinimide and azobisisobutyronitrile, stirred under reflux and then subjected to purification to obtain 2-(4-fluoro-3-nitrophenyl)-1,3-oxazole as a colorless solid. F-: 208.

Reference Example H3: Under ice-cooling, TEA and sulfur trioxide-pyridine complex were added to a dichloromethane/DMSO mixed solution of 3-quinolin-6-ylpropan-1-ol, stirred and then subjected to purification to obtain 3-quinolin-6-ylpropanal as a brown oil. N2: 2.87 - 2.93 (2 H, m), 3.15 (2 H, t), 7.39 (1 H, dd), 7.57 (1 H, dd), 7.61 (1 H, s), 8.05 (1 H, d), 8.08 - 8.12 (1 H, m), 8.87 (1 H, dd), 9.86 (1 H, t).

Reference Example H4: Under ice-cooling, 2-methyl-2-butene, sodium dihydrogenphosphate and sodium chlorite were added to a tert-butanol/water mixed solution of 3-quinolin-6-ylpropanal and stirred at the same temperature for 2 hours. This was adjusted to pH 5 to 6 and extracted with chloroform, and then the solvent was evaporated to obtain 3-quinolin-6-ylpropionic acid as a colorless solid. F+: 202.

Reference Example I1: A THF solution of N,N-diethyl-4-fluoro-3-nitrobenzamide was mixed with 1.0 M borane/THF solution and stirred under reflux. The reaction solution was ice-cooled, mixed with methanol and then stirred. After evaporation of the solvent, the resulting residue was mixed with 6 M hydrochloric acid and stirred at 100°C. After spontaneous cooling, the reaction solution was alkalized and extracted with ethyl acetate. The resulting organic layer was extracted with 1 M hydrochloric acid, and the extract was alkalized and extracted with chloroform. By evaporating the solvent, N,N-diethyl-N-(4-fluoro-3-nitrobenzyl)amine was obtained as a yellow oil. F+: 227.

Reference Example I2: Under ice-cooling, a TFA solution of ethyl 4-oxo-4-(2-oxoindolin-5-yl)butyrate was mixed with triethylsilane and stirred at 45°C and then at room temperature. By purifying the resulting product, ethyl 4-(2-oxoindolin-5-yl)butyrate was obtained as a colorless solid. F+: 248.

Reference Example I3: Under ice-cooling, lithium aluminum hydride was added to a THF solution of ethyl

3-quinolin-7-ylpropionate and stirred at the same temperature for 4 hours. Water was added to the reaction solution, and the thus formed precipitate was removed by filtration. The filtrate was concentrated, and the resulting residue was subjected to the separation of layers using chloroform and water. By purifying the product from the organic layer, 3-quinolin-7-ylpropan-1-ol was obtained as a colorless oil. F+: 230.

Reference Example I4: Hydrogenation was carried out under ordinary pressure and at ordinary temperature, by adding 10% palladium-carbon (Pd-C) to an ethanol solution of 6-(3-hydroxy-1-propinyl)quinoline. When theoretical amount of hydrogen was absorbed, the catalyst was removed through celite pad. After evaporation of the solvent, the resulting residue was purified by a silica gel column chromatography (to be referred to as SCC hereinafter) to obtain 3-quinolin-6-ylpropan-1-ol as a colorless oil. N2: 1.95 - 2.05 (3 H, m), 2.92 (2 H, t), 3.73 (2 H, t), 7.38 (1 H, dd), 7.58 (1 H, dd), 7.61 (1 H, s), 8.03 (1 H, d), 8.09 (1 H, dd), 8.86 (1 H, dd).

Reference Example J1: An ethanol solution of 2-bromo-1-(4-chloro-3-nitrophenyl)ethanone was mixed with thioacetamide, stirred and then subjected to purification to obtain 4-(4-chloro-3-nitrophenyl)-2-methyl-1,3-thiazole as a colorless solid. F+: 255.

Reference Example J2: A benzene solution of 4-fluoro-3-nitrobenzoic acid was mixed with thionyl chloride and stirred under reflux. After evaporation of the solvent, the resulting residue was dissolved in dichloromethane, added to a dichloromethane solution of 2-amino-2-methylpropan-1-ol under ice-cooling and then stirred. The thus formed precipitate was removed by filtration and washed with chloroform. The solvent was evaporated from the resulting filtrate and the residue was mixed with thionyl chloride and stirred. The reaction solution was mixed with diethyl ether to remove diethyl ether-solubilized fraction by decantation and then diluted with chloroform. By purifying the product from the organic layer, 2-(4-fluoro-3-nitrophenyl)-4,4-dimethyl-4,5-dihydro-1,3-oxazole was obtained as a yellow oil. N2: 1.39 (6 H, s), 4.16 (2 H, s).

Reference Example J3: An N-(1,1-dimethoxyethyl)-N,N-dimethylamine solution of 4-chloro-3-nitrobenzamide was stirred at 100°C. After evaporation of the solvent, hydroxylamine hydrochloride, 1 M sodium hydroxide aqueous solution, 1,4-dioxane and acetic acid were added to the resulting residue and stirred at room temperature and then at 90°C. After evaporation of the solvent, the resulting residue was mixed with 1 M sodium hydroxide aqueous solution, extracted with chloroform and then purified to obtain 5-(4-chloro-3-nitrophenyl)-3-methyl-1,2,4-oxadiazole as an orange solid. F+: 240.

Reference Example K: Under ice-cooling, phosphorus tribromide was added dropwise to a dichloromethane solution of 3-quinolin-6-ylpropan-1-ol, and the mixture was slowly warmed up to room temperature and then stirred under reflux. After spontaneous cooling, this was purified to obtain 6-(3-bromopropyl)quinoline as a colorless oil. N2: 2.27 (2 H, qui), 2.99 (2 H, t), 3.43 (2 H, t), 7.40 (1 H, dd), 7.59 (1 H, dd), 7.63 (1 H, s), 8.06 (1 H, d), 8.13 (1 H, dd), 8.88 (1 H, dd).

Reference Example L: A diethylamine solution of 6-bromoquinoline and propargyl alcohol was mixed with (bistriphenylphosphine)palladium(II) chloride and cuprous iodide and stirred at 45°C. By purifying the thus formed substance from the reaction solution, 6-(3-hydroxy-1-propinyl)quinoline was obtained as a colorless solid. F+: 184.

Reference Example M1: Concentrated sulfuric acid was added to an ethanol solution of 4-chloro-2-methoxy-5-nitrobenzoic acid, and the mixture was stirred under reflux and then purified to obtain ethyl 4-chloro-2-methoxy-5-nitrobenzoate. F+: 260.

Reference Example M2: Potassium carbonate and propyl iodide were added to a DMF solution of 4-bromo-2-methyl-5-nitrobenzoic acid, and the mixture was stirred and then purified to obtain propyl 4-bromo-2-methyl-5-nitrobenzoate. E: 301, 303.

Reference Example N: Under ice-cooling, ethyl succinyl chloride was added to a dichloroethane suspension of indolin-2-one and aluminum chloride, and the mixture was stirred at room temperature and then at 50°C. After spontaneous cooling, the reaction solution was poured into ice water, and the thus formed precipitate was collected by filtration to obtain ethyl 4-oxo-4-(2-oxoindolin-5-yl)butyrate as brown solid. F+: 262.

Reference Example O: A dimethoxyethane solution of 6-bromoindolin-2-one was mixed with tetrakistriphenylphosphine palladium and stirred. The reaction solution was mixed with 3-furylboronic acid and an aqueous solution of sodium carbonate and stirred under reflux. By purifying the thus formed product, 6-(3-furyl)indolin-2-one was obtained as a pink solid. F+: 200.

Reference Example P: Pyridine and methanesulfonyl chloride were added to a dichloromethane solution of ethyl 3-(5-amino-2-methoxyphenyl)propionate, and the mixture was stirred and then purified to obtain ethyl 3-{2-methoxy-5-[(methylsulfonyl)amino]phenyl}propionate as a brown solid. F+: 302.

Reference Example Q: A methanol solution of ethyl 3-{2-methoxy-5-[(methylsulfonyl)amino]phenyl}propionate and TEA was mixed with 90% acrolein and stirred. After evaporation of the solvent, the resulting residue was dissolved in dichloromethane, mixed with trifluoromethanesulfonic acid, stirred and then subjected to purification to obtain ethyl 3-[6-methoxy-1-(methylsulfonyl)-1,2-dihydroquinolin-7-yl]propionate as colorless solid. N2: 1.26 (3 H, t), 3.83 (3 H, s).

Reference Example R: An ethanol solution of ethyl 3-[6-methoxy-1-(methylsulfonyl)-1,2-dihydroquinolin-7-yl]propionate was mixed with potassium hydroxide aqueous solution and stirred. After adding 1 M hydrochloric acid to the reaction solution, the solvent was evaporated. Ethanol and concentrated sulfuric acid were added to the resulting residue, and the mixture was stirred under reflux. After spontaneous cooling, the reaction solution was diluted with chloroform and water and alkalized, and then the reaction solution was separated into two layers and the product was purified from the resulting organic layer to obtain ethyl 3-(6-methoxyquinolin-7-yl)propionate as a colorless solid. N2: 1.22 (3 H, t), 4.12 (2 H, q).

Reference Example S: Ethyl acetate and saturated sodium bicarbonate aqueous solution were added to 4-(4-chloro-3-nitrophenyl)-1,3-thiazole-2-amine hydrobromide and separated. The solvent was evaporated from the organic layer, the resulting residue was dissolved in DMF and added to a DMF solution of isoamyl nitrite at 70°C, and then the mixture was stirred at the same temperature. By purifying the thus formed material, 4-(4-chloro-3-nitrophenyl)-1,3-thiazole was obtained as a colorless oil. F+: 241.

Reference Example T: A carbon tetrachloride solution of ethyl 4-bromo-2-methylbenzoate was mixed with N-bromosuccinimide and azobisisobutyronitrile, and the mixture was stirred under reflux and then purified to obtain ethyl 4-bromo-2-(dibromomethyl)benzoate as a colorless solid. N2: 1.42 (3 H, t), 4.40 (2 H, q), 7.99 (1 H, s).

Reference Example U: Under ice-cooling, ethyl 4-bromo-2-(dibromomethyl)benzoate and potassium nitrate were added to concentrated sulfuric acid, and the mixture was stirred and then purified to obtain ethyl 4-bromo-2-formylbenzoate as a colorless oil. E: 256, 258.

Reference Example V: Under ice-cooling, 4-bromo-2-methylbenzaldehyde and potassium nitrate were added to concentrated sulfuric acid, and the mixture was stirred. The reaction solution was poured into ice water, and the thus formed precipitate was collected by filtration and then washed to obtain 4-bromo-2-methyl-5-nitrobenzaldehyde as a brown solid. N1: 2.74 (3 H, s), 10.24 (1 H, s).

Reference Example W: Meldrum's acid was added to methyl orthoformate and stirred at 100°C for 10 minutes. The reaction solution was mixed with 4-bromo-3-methoxyaniline and stirred under reflux and then under spontaneous cooling. The thus formed precipitate was collected by filtration and then washed to obtain 5-{[(4-bromo-3-methoxyphenyl)amino]methylene}-2,2-dimethyl-1,3-dioxane-4,6-dione as a brown solid. F+: 355, 357.

Reference Example X: Diphenyl ether was added to DOW THERM (mfd. by Fluka), and the mixture was heated to 270°C. This was mixed with 5-{[(4-bromo-3-methoxyphenyl)amino]methylene}-2,2-dimethyl-1,3-dioxane-4,6-dione, stirred at the same temperature, spontaneously cooled to 40°C and then mixed with petroleum ether, and the thus formed precipitate was collected by filtration to obtain 6-bromo-7-methoxyquinolin-4(1H)-one as a brown solid. F+: 253, 255.

Reference Example Y: A thionyl chloride solution of 6-bromo-7-methoxyquinolin-4(1H)-one was mixed with DMF and stirred under reflux. The solvent was evaporated, the resulting residue was mixed with chloroform and toluene, and the solvents were again evaporated. By crystallizing the resulting residue from diethyl ether, 6-bromo-4-chloro-7-methoxyquinoline was obtained as a colorless solid. F+: 271, 273.

Reference Example Z: Under ice-cooling, phthalimide, triphenylphosphine and diethyl azodicarboxylate were added to a THF/DMF mixed solution of (1,1-dioxidotetrahydro-2H-thiopyran-4-yl)methanol, and the mixture was stirred at the same temperature and then subjected to purification to obtain 2-[(1,1-dioxidotetrahydro-2H-thiopyran-4-yl)methyl]isoindoline-1,3-dione as colorless solid. F+: 294.

Reference Example AA1: tert-Butyl 2-(1-oxidopyridin-4-yl)ethylcarbamate was added to 4 M hydrogen chloride/ethyl acetate solution and stirred at room temperature. The thus formed precipitate was collected by filtration and washed with ethyl acetate to obtain 2-(1-oxidopyridin-4-yl)ethylamine as a yellow solid. F+: 139.

Reference Example AA2: Hydrazine monohydrate was added to an ethanol solution of 2-[(1,1-dioxidotetrahydro-2H-thiopyran-4-yl)methyl]isoindoline-1,3-dione, and the mixture was stirred under reflux. After spontaneous cooling, the thus formed precipitate was removed through celite, and the filtrate was concentrated. Chloroform was added to the resulting residue, the insoluble matter was removed by filtration and then the filtrate was concentrated to obtain (1,1-dioxidotetrahydro-2H-thiopyran-4-yl)methylamine as a brown oil. F+: 164.

Reference Example BB: Dimethyl malonate was slowly added dropwise to a DMSO suspension of 60% NaH, and then the mixture was stirred at 100°C. After cooling down to room temperature, this was mixed with N-(2,5-dichloro-4-nitrophenyl)acetamide and stirred at the same temperature and then at 100°C. The thus formed product was purified and then crystallized from ethyl acetate to obtain dimethyl [5-(acetylamino)-4-chloro-2-nitrophenyl]malonate as a colorless solid. F+: 344.

Reference Example CC1: Anhydrous lithium chloride and water were added to a DMSO solution of dimethyl [5-(acetylamino)-4-chloro-2-nitrophenyl]malonate, and the mixture was stirred at 100°C. After spontaneous cooling, the reaction solution was poured into a mixed solution of ethyl acetate and saturated brine and extracted with ethyl acetate. The resulting organic layer was washed and then concentrated, and the thus obtained crude crystals were recrystallized from methanol to obtain methyl [5-(acetylamino)-4-chloro-2-nitrophenyl]acetate as a colorless solid. F: 286.

Reference Example CC2: A 6 M hydrochloric acid solution of diethyl (4-formyl-2-nitrophenyl)malonate was

stirred under reflux. The reaction solution was ice-cooled, and then the thus formed precipitate was collected by filtration and washed to obtain (4-formyl-2-nitrophenyl)acetic acid. F+: 210.

Reference Example DD: Reduced iron was added to an acetic acid solution of methyl [5-(acetylamino)-4-chloro-2-nitophenyl]acetate, and the mixture was stirred at 100°C, After spontaneous cooling, the reaction solution was filtered through celite and washed with DMF. The filtrate was concentrated and then mixed with water, and the thus formed precipitate was collected by filtration and washed with water to obtain N-(6-chloro-2-oxoindolin-5-yl)acetamide as a colorless solid. F+: 225.

Reference Example EE: Tetrahydrofuran-2-ylmethylamine was added to a toluene solution of quinoline-7-carbaldehyde, and the mixture was stirred under reflux using a Dean-Stark apparatus. The solvent was evaporated, and the resulting residue was dissolved in methanol, mixed with sodium borohydride and then stirred. After evaporation of the solvent, the resulting residue was dissolved in THF, mixed with di-tert-butyl dicarbonate and then stirred at 70°C. After evaporation of the solvent, the resulting residue was purified by SCC to obtain tert-butyl quinolin-7-ylmethyl(tetrahydrofuran-2-ylmethyl)carbamate as a colorless oil. F+: 343.

Reference Example FF: Diethylamine and sodium triacetoxyborohydride were added to a 1,2-dichloroethane solution of ethyl 3-bromo-2-formylbenzoate, and the mixture was stirred. After purification, ethyl 3-bromo-2-(diethylaminomethyl)benzoate was obtained as a colorless oil. F+: 314, 316.

The reference example compounds shown in Tables 2 to 5 were obtained in the same manner as the case of the aforementioned reference examples.

Example 1: Under ice-cooling, benzoyl chloride (0.3 ml) was added to a chloroform (25 ml) solution of 6-[2-(1H-1,2,3-triazol-1-yl)ethoxy]quinoline N-oxide (510 mg), and the mixture was stirred at the same temperature for 30 minutes. Next, indolin-2-one (265 mg) was added thereto and heated at 90°C under reflux for 8 hours. After spontaneous cooling, saturated sodium bicarbonate aqueous solution and ethyl acetate were added thereto and stirred for 30 minutes. The thus formed precipitate was collected by filtration and washed with ethyl acetate. On the other hand, organic layer of the mother liquor was concentrated, and the thus formed precipitate was collected by filtration and washed with ethyl acetate. The two precipitates were combine and recrystallized from ethanol to obtain 111 mg of 3-{6-[2-(1H-1,2,3-triazol-1-yl)ethoxy]quinolin-2(1H)-ylidene}indolin-2-one as a red solid.

Example 2: A dichloromethane (20 ml) solution of ethyl quinoline-7-carboxylate (3.07 g) was mixed with m-chloroperbenzoic acid (3.3 g) and stirred at room temperature for 1 hour. After evaporating the solvent, the resulting residue was collected by filtration and washed with ethyl acetate. The thus obtained solid matter was dissolved in acetic anhydride (30 ml), mixed with indolin-2-one (3.1 g) and then stirred at 55°C for 12 hours. After evaporation of the solvent, ethanol was added and the thus formed precipitate was collected by filtration. By recrystallizing the thus obtained crude crystals from ethanol, 65 mg of ethyl 2-(2-oxoindolin-3-ylidene)-1,2-dihydroquinoline-7-carboxylate was obtained as a red solid.

Example 3: By the same method of Example 1, (a) 234 mg of 2-(2-oxoindolin-3-ylidene)-1,2-dihydroquinoline-4-carbaldehyde was obtained as a red solid from indolin-2-one (2.11 g) and quinoline-4-carbaldehyde 1-oxide (2.12 g). The crystallization mother liquor was concentrated, the resulting residue was purified by an SCC (elution with chloroform-methanol), and then the thus obtained solid matter was collected by filtration and recrystallized from ethanol to obtain (b) 55 mg of 3-(4-diethoxymethylquinolin-2(1H)-ylidene)indolin-2-one as a brown solid.

Example 4: 3-[6-(2-Bromoethoxy)quinolin-2(1H)-ylidene]indolin-2-one (1.86 g) was suspended in acetonitrile (100 ml), and the suspension was mixed with morpholine (2.11 g) and stirred at 80°C for 4 hours. After spontaneous cooling, the reaction solution was mixed with saturated sodium bicarbonate aqueous solution and saturated brine and extracted with chloroform. The resulting organic layer was dried with anhydrous sodium sulfate, and then the solvent was evaporated. The resulting residue was purified by an SSC (elution with methanol-ethylacetate-28% aqueous ammonia), and the thus obtained solid matter was recrystallized from ethanol to obtain 960 mg of 3-[6-(2-morpholin-4-ylethoxy)quinolin-2(1H)-ylidene]indolin-2-one as a red solid.

Example 5: Acetic acid (0.99 ml) was added to a dichloroethane (35 ml) solution of 2-(2-oxoindolin-3-ylidene)-1,2-dihydroquinoline-6-carbaldehyde (0.5 g) and 2-morpholin-4-ylethylamine (0.91 ml), and the mixture was stirred at room temperature for 2 hours. The reaction solution was mixed with sodium triacetoxyborohydride (1.1 g) and stirred at room temperature for 13 hours. The reaction solution was mixed with saturated sodium bicarbonate aqueous solution and extracted with dichloroethane. The resulting organic layer was washed with water and saturated brine and dried with anhydrous magnesium sulfate, and then the solvent was evaporated. After purifying the resulting residue by an SSC (elution with chloroform), the thus obtained solid matter was collected by filtration and washed with ethyl acetate to obtain 168 mg of 3-(6-{[(2-morphilin-4-ylethyl)amino]methyl}quinolin-2(1H)-ylidene)indolin-2-one as an orange solid.

Example 6: Titanium tetraisopropoxide (0.68 ml) was added to a dichloroethane (3 ml) solution of 2-(2-oxoindolin-3-ylidene)-1,2-dihydroquinoline-6-carbaldehyde (0.6 g), N-(2-methoxyethyl)-N-methylamine (0.89 ml), and the mixture was stirred at room temperature for 1 hour. After ice-cooling, the reaction solution was mixed with sodium triacetoxyborohydride (1.32 g) and stirred at room temperature for 1.5 hours. The reaction solution was mixed with saturated sodium bicarbonate aqueous solution and extracted with dichloroethane. The resulting organic layer was

washed with water and saturated brine and dried with anhydrous magnesium sulfate, and then the solvent was evaporated. After purifying the resulting residue by an SSC (elution with chloroform), the thus obtained solid matter was collected by filtration and washed with ethyl acetate to obtain 114 mg of 3-(6-{[N-(2-methoxyethyl)-N-methylamino]methyl}quinolin-2(1H)-ylidene)indolin-2-one as a red solid.

Example 7: Pyrrolidine (64 mg) was added to a mixed solution of ethanol (3 ml) and chloroform (3 ml) containing 3-[6-(oxiran-2-ylmethoxy)quinolin-2(1H)-ylidene]indolin-2-one (100 mg), and the mixture was stirred at 40°C for 1.5 hours. After spontaneous cooling, the solvent was evaporated. After purifying the resulting residue by an SSC (elution with chloroform-methanol-28% aqueous ammonia), the thus obtained solid matter was recrystallized from 2-propanol to obtain 9 mg of 3-[6-(2-hydroxy-3-pyrrolidin-1-ylpropoxy)quinolin-2(1H)-ylidene]indolin-2-one as an orange solid.

Example 8: Morpholine (0.088 ml) was added to a DMF (10ml) solution of {[2-(2-oxoindolin-3-ylidene)-1,2-dihydroquinolin-6-yl]oxy}acetic acid (280 mg), 1-hydroxybenzotriazole (79 mg) and 1-ethyl-3-(3-dimethylaminopropyl)carbodiimide hydrochloride (225 mg), and the mixture was stirred at room temperature for 3 days. The reaction solution was poured into water, and the thus formed precipitate was collected by filtration and then washed with successive water and ethanol to obtain 253 mg of 3-[6-(2-morpholin-4-yl-2-oxoethoxy)quinolin-2(1H)-ylidene]indolin-2-one as a red solid.

Example 9: Under ice-cooling, TEA (0.3 ml) and bromoacetic acid bromide (0.15 ml) were added to an acetonitrile (10 ml) suspension of 3-[6-aminoquinolin-2(1H)-ylidene]indolin-2-one (142 mg), and the mixture was stirred at room temperature. This was mixed with saturated sodium bicarbonate aqueous solution and chloroform and stirred at room temperature for 30 minutes. The thus formed precipitate was collected by filtration and washed with chloroform to obtain a red solid matter (196 mg). The thus obtained red solid matter was suspended in acetonitrile (20 ml), mixed with morpholine (413 mg) and then stirred at 70°C for 2 hours. After spontaneous cooling, the reaction solution was mixed with saturated sodium bicarbonate aqueous solution and acetonitrile and stirred at room temperature for 30 minutes. The thus formed precipitate was collected by filtration and washed with acetonitrile to obtain a reddish black solid matter. The thus obtained reddish black solid matter was purified by an SCC (elution with methanol-ethyl acetate-chloroform-28% saturated aqueous ammonia), and the thus obtained solid matter was washed with ethanol to obtain 72 mg of 2-morpholin-4-yl-N-[2-(2-oxoindolin-3-ylidene)-1,2-dihydroquinolin-6-yl]acetamide as a red solid.

Example 10: A DMF (50 ml) solution of 3-[6-nitroquinolin-2(1H)-ylidene]indolin-2-one (1.13 g) was mixed with 10% Pd-C (57 mg), and the mixture was stirred under an atmosphere of hydrogen at room temperature for 17 hours. The reaction solution was filtered through celite, and the filtrate was mixed with saturated brine and extracted with ethyl acetate. The organic layer was dried with anhydrous sodium sulfate and then the solvent was evaporated. The resulting residue was purified by an SCC (elution with methanol-chloroform-ethyl acetate-28% aqueous ammonia), and the thus obtained solid matter was washed with boiling ethanol to obtain 463 mg of 3-[6-aminoquinolin-2(1H)-ylidene]indolin-2-one as a red solid.

Example 11: A chloroform (20 ml) solution of 3-[6-(2-(thiomorphin-4-ylethoxy)quinolin-2(1H)-ylidene)indolin]-2-one (690 mg) was mixed with m-chloroperbenzoic acid (414 mg) and stirred at room temperature for 2 hours. The thus formed precipitate was collected by filtration and recrystallized from methanol to obtain 12 mg of 3-{6-[2-(1,4-dioxidothiomorpholin-4-yl)ethoxy]quinolin-2(1H)-ylidene}indolin-2-one as an orange solid.

Example 12: Under ice-cooling, an ethanol (1 ml) solution of tert-butyl 4-{[2-(2-oxoindolin-3-ylidene)-1,2-dihydroquinoline-6-yl]carbonyl}piperadin-1-ylcarboxylate (155 mg) was mixed with 4 M hydrogen chloride/ethyl acetate solution (5 ml) and stirred at the same temperature for 2 hours. After concentration of the reaction solution, the resulting residue was collected by filtration and washed with hot ethanol to obtain 122 mg of 3-[6-(piperadine-1-carbonyl)quinolin-2(1H)-ylidene]indolin-2-one hydrochloride as an orange solid.

Example 13: Hydrazine hydrochloride (6.5 mg) was added to an ethanol (10 ml) solution of 2-{[6-(2-methoxyethoxy)-2-(2-oxoindolin-3-ylidene)-1,2-dihydroquinolin-5-yl]methyl}isoindoline-1,3-dione (20 mg), and the mixture was stirred at 100°C for 5 hours. After spontaneous cooling, the resulting filtrate through celite was concentrated. The resulting residue was dissolved in ethanol (2 ml), mixed with 4 M hydrogen chloride/ethyl acetate solution under ice cooling, and then stirred at the same temperature for 15 minutes. After evaporation of the solvent, the resulting residue was collected by filtration and washed with hot 2-propanol to obtain 5 mg of 3-[5-aminomethyl-6-(2-methoxyethoxy)quinolin-2(1H)-ylidene]indolin-2-one hydrochloride as a red solid.

Example 14: An methanol (10 ml) suspension of 2-(2-oxoindolin-3-ylidene)-1,2-dihydroquinoline-6-ylmethyl benzoate (200 mg) was mixed with 1 M sodium hydroxide aqueous solution (4 ml) and stirred under heating at 70°C for 15 minutes. After spontaneous cooling, this was mixed with brine and extracted with ethyl acetate. The resulting organic layer was dried with anhydrous sodium sulfate and then concentrated, and the thus obtained residue was purified by an SCC (elution with methanol-ethyl acetate-28% aqueous ammonia). By recrystallizing the thus obtained solid matter from ethanol, 24 mg of 3-(6-hydroxymethylquinolin-2(1H)-ylidene)indolin-2-one was obtained as a red solid.

Example 15: A methanol (5 ml)/THF (10 ml) mixed solution of 3-[6-(piperidin-4-ylmethoxy)quinolin-2(1H)-ylidene]indolin-2-one hydrochloride (190 mg) was mixed with 35% formalin (0.08 ml) and sodium cyanoborohydride (35 mg) and stirred at room temperature for 3 hours. The reaction solution was mixed with saturated sodium bicarbonate aqueous solution, and the thus formed precipitate was collected by filtration and washed with water. The thus obtained solid matter was purified by an SCC (elution with chloroform-ethyl acetate-methanol-28% aqueous ammonia). The thus obtained solid matter was collected by filtration and washed with hot ethanol to obtain 30 mg of 3-{6-[(1-methylpiperidin-4-yl)methoxy]quinolin-2(1H)-ylidene}indolin-2-one as a red solid.

Example 16: A xylene (15 ml) suspension of 3-[6-aminoquinolin-2(1H)-ylidene]indolin-2-one (103 mg) was mixed with 3-pyridyl isocyanate (162 mg) and heated at 130°C under reflux. The thus formed precipitate was collected by filtration, heated under reflux in methanol (25 ml) and then filtered while hot, thereby obtaining 48 mg of 1-[2-(2-oxoindolin-3-ylidene)-1,2-dihydroquinolin-6-yl]-3-(pyridin-3-yl)urea as a red solid.

Example 17: A DMF (10 ml) solution of ethyl quinolin-2-ylacetate (860 mg) was mixed with 60% NaH (320 mg) and stirred at room temperature for 10 minutes. Next, this was mixed with ethyl 4-fluoro-3-nitrobenzoate (746 mg) under ice-cooling and then stirred at the same temperature for 30 minutes. The reaction solution was poured into ice water, acidified by adding 1 M hydrochloric acid aqueous solution and then extracted with ethyl acetate. The resulting organic layer was washed with brine, dried over anhydrous sodium sulfate and then concentrated. The resulting residue was mixed with acetic acid (20 ml) and reduced iron (800 mg) and stirred at 100°C for 1 hour. The insoluble matter was removed by filtration and washed with DMF. The mother liquor was mixed with ethyl acetate and washed with water and brine in that order, and then the organic layer was dried over anhydrous sodium sulfate and concentrated. By washing the resulting residue with methanol, 274 mg of ethyl 3-[quinolin-2(1H)-ylidene]indolin-2-one-carboxylate was obtained as a red solid.

Example 18: Dimethylamine hydrochloride (1.6 g) was added to a pyridine (15 ml) solution of ethyl (5-fluoro-2-nitrophenyl)(quinolin-2(1H)-ylidene)acetate, and the mixture was stirred at 100°C for 4 hours. After spontaneous cooling, this was diluted with ethyl acetate and washed with water and brine. The resulting organic layer was dried over anhydrous magnesium sulfate, and then the solvent was evaporated. The resulting residue was dissolved in acetic acid (10 ml), mixed with reduced iron (200 mg) and then stirred at 100°C for 2 hours. After spontaneous cooling, the reaction solution was filtrated through celite and washed with methanol. After concentration of the filtrate, the resulting residue was dissolved in chloroform and washed with water and brine. The resulting organic layer was dried with anhydrous magnesium sulfate, and then the solvent was evaporated. The resulting residue was purified by an SCC (elution with ethyl acetate-hexane), and the thus obtained solid matter was collected by filtration and washed with ethyl acetate to obtain 15 mg of 5-dimethylamino-3-quinolin-2(1H)-ylideneindolin-2-one as a red solid.

Example 19: Quinoline 1-oxide (214 mg) was added to an acetic anhydride (10 ml) solution of 6-methoxyindolin-2-one (200 mg), and the mixture was stirred at 50°C for 5 hours. After spontaneous cooling, this was poured into ice water and extracted with ethyl acetate. The resulting organic layer was washed with successive, saturated sodium bicarbonate aqueous solution, water, and brine and dried over anhydrous magnesium sulfate, and then the solvent was evaporated. The resulting residue was purified by an SCC (elution with chloroform) and then recrystallized from diethyl ether. The thus obtained crystals were collected by filtration and washed with chloroform to obtain 30 mg of 6-methoxy-3-quinolin-2(1H)-ylideneindolin-2-one as a red solid.

Example 20: Under ice-cooling, 60% NaH (96 mg) was added to a THF (10 ml) solution of ethyl quinolin-2-ylacetate (516 mg) and 1-(4-chloro-3-nitrophenyl)ethanone (400 mg), and the mixture was stirred at room temperature for 2 hours. The reaction solution was diluted with ethyl acetate and washed with water and brine. The resulting organic layer was dried with anhydrous magnesium sulfate, and then the solvent was evaporated. The thus obtained residue was dissolved in methanol (10 ml), mixed with hydroxylamine hydrochloride (168 mg) and then stirred at 50°C for 18 hours. After evaporation of the solvent, the resulting residue was dissolved in acetic acid (20 ml), mixed with reduced iron (600 mg) and then stirred at 50°C for 18 hours. The reaction solution was subjected to celite filtration and washed with ethyl acetate. The resulting filtrate was washed with successive, water, 1 M sodium hydroxide aqueous solution, and brine, the resulting organic layer was dried over anhydrous magnesium sulfate, and then the solvent was evaporated. The resulting residue was collected by filtration and washed with 2-propanol. By recrystallizing the thus obtained crude crystals from DMF, 250 mg of 6-(1-methoxyiminoethyl)-3-quinolin-2(1H)-ylideneindolin-2-one was obtained as a red solid.

Example 21: Reduced iron (50 mg) was added to an acetic acid (5 ml) solution of 6-acetyl-1-hydroxy-3-quinolin-2(1H)-ylideneindolin-2-one (96 mg), and the mixture was stirred at 100°C for 1 hour. The reaction solution was filtered through celite and the filtrate was concentrated. By recrystallizing the resulting residue from DMF, 35 mg of 6-acetyl-3-quinolin-2(1H)-ylideneindolin-2-one was obtained as an orange solid.

Example 22: A methanol (5 ml) solution of acetic 2-oxo-3-quinolin-2(1H)-ylideneindoline-5-carboxylic anhydride (234 mg) was mixed with 1 M sodium hydroxide aqueous solution (2 ml), and the mixture was stirred at room temperature for 10 minutes. The reaction solution was acidified by adding 1 M hydrochloric acid, and then the thus formed precipitate was collected by filtration and washed with diethyl ether and water. By recrystallizing the thus obtained crude crystals from DMF, 65 mg of 2-oxo-3-quinolin-2(1H)-ylideneindoline-5-carboxylic acid was obtained as

an orange solid.

Example 23: Ethyl 4-(2-oxo-3-quinolin-2(1H)-ylideneindolin-5-yl)butyrate (350 mg) was suspended in 6 M hydrochloric acid (15 ml) and stirred under reflux for 5 hours. After spontaneous cooling, the thus formed precipitate was collected by filtration and washed with water to obtain 97 mg of 4-(2-oxo-3-quinolin-2(1H)-ylideneindolin-5-yl) butyric acid as an orange solid.

Example 24: An acetic acid (10 ml) solution of 5-benzyloxy-3-quinolin-2(1H)-ylideneindolin-2-one (183 mg) was mixed with 5% Pd-C (100 mg), and the mixture was subjected to 18 hours of hydrogenation at room temperature under 4 atmospheric pressure. The reaction mixture was filtered through celite, and the filtrate was concentrated. The thus obtained solid matter was collected by filtration and washed with methanol to obtain 68 mg of 5-hydroxy-3-quinolin-2(1H)-ylideneindolin-2-one as a red solid.

Example 25: To a DMF (20 ml) solution of benzyl 2-oxo-3-quinolin-2(1H)-ylideneindoline-6-carboxylate (120 mg) was added 5% Pd-C (50 mg), and the mixture was subjected to 3 days of hydrogenation at room temperature under 1 atmospheric pressure. The reaction solution was mixed with DMF (80 ml), filtered through celite while hot, and the filtrate was concentrated. The thus obtained solid matter was collected by filtration and washed with methanol to obtain 44 mg of 2-oxo-3-quinolin-2(1H)-ylideneindoline-6-carboxylic acid as a red solid.

Example 26: TFA (5 ml) was added to a mixed solution of dichloromethane (5 ml) and THF (5 ml) containing tert-butyl 2-(2-oxo-3-quinolin-2(1H)-ylideneindolin-6-yl)-1H-pyrrole-1-carboxylate (93 mg), and the mixture was stirred at room temperature for 18 hours. After evaporation of the solvent, methanol was added to the resulting residue, and the thus formed crystals were collected by filtration to obtain 56 mg of 6-(1H-pyrrol-2-yl)-3-quinolin-2(1H)-ylideneindolin-2-one as a red solid.

Example 27: Thioacetamide (90 mg) was added to a DMF (10 ml) solution of 5-(2-chloroacetyl)-3-quinolin-2 (1H)-ylideneindolin-2-one (337 mg), and the mixture was stirred at 100°C for 1 hour. The reaction solution was poured into water, and the thus formed precipitate was collected by filtration and washed with water and ethanol. The thus obtained solid matter was dissolved in a chloroform-methanol mixed solution and washed with water and brine, the resulting organic layer was dried with anhydrous magnesium sulfate, and then the solvent was evaporated. By recrystallizing the resulting residue from DMF, 68 mg of 5-(2-methyl-1,3-thiazol-4-yl)-3-quinolin-2(1H)-ylideneindolin-2-one was obtained as a red solid.

Example 28: Imidazole (144 mg) was added to a DMF (20 ml) solution of 5-(4-chlorobutanoyl)-3-quinolin-2 (1H)-ylideneindolin-2-one (350 mg), and the mixture was stirred at 55°C for 5 days. After spontaneous cooling, the reaction solution was poured into ice water, and the thus formed precipitate was collected by filtration and washed with water. The just described precipitate was purified by an SCC (elution with chloroform-methanol), and the thus obtained solid matter was collected by filtration and washed with diethyl ether to obtain 61 mg of 5-(4-hydroxybutanoyl)-3-quinolin-2(1H)-ylideneindolin-2-one as an orange solid.

Example 29: Ethoxylamine hydrochloride (98 mg) was added to a methanol (5 ml) solution of ethyl (4-formyl-2-nitrophenyl)[6-(2-morpholin-4-ylethoxy)quinolin-2(1H)-ylidene]acetate (354 mg), and the mixture was stirred at room temperature for 1 hour. After evaporation of the solvent, the resulting residue was dissolved in acetic acid (10 ml), mixed with reduced iron (200 mg) and then stirred at 100°C for 1 hour. After spontaneous cooling, this was mixed with chloroform-2-propanol (3:1) mixed solution and 1 M sodium hydroxide aqueous solution, filtrated through celite and then washed with chloroform. The resulting filtrate was subjected to the separation of layers, and the organic layer was washed with water and then with brine. The organic layer was dried with anhydrous magnesium sulfate, and then the solvent was evaporated. The resulting residue was purified by an SCC (elution with chloroform-methanol). The thus obtained solid matter was dissolved in methanol (5 ml), mixed with 4 M hydrogen chloride/ethyl acetate solution (0.25 ml) and then stirred at room temperature for 10 minutes. The thus formed precipitate was collected by filtration and washed with methanol to obtain 58 mg of 3-[6-(2-morpholin-4-ylethoxy)quinolin-2(1H)-ylidene]-2-oxoindoline-6-carbaldehyde O-ethyloxime hydrochloride as a red solid.

Example 30: Under ice-cooling, 60% NaH (80mg) was added to a DMF (5 ml) solution of ethyl quinolin-2-ylacetate (215 mg) and 1-(4-chloro-3-nitrophenyl)ethanone (200 mg), and the mixture was stirred at the same temperature for 1 hour. The reaction solution was poured into water and acidified using 1 M hydrochloric acid, and then the thus formed precipitate was collected by filtration and washed with water. The thus obtained solid matter was dissolved in acetic acid (10 ml), mixed with 5% Pd-C (50 mg) and then subjected to 18 hours of hydrogenation at room temperature under 3.5 atmospheric pressure. The reaction mixture was filtered through celite and washed with DMF, and the filtrate was concentrated. The thus obtained residue was collected by filtration and washed with ethanol. By recrystallizing the thus obtained crude crystals from DMF, 70 mg of 6-acetyl-1-hydroxy-3-quinolin-2(1H)-ylideneindolin-2-one.

The Example compounds described in the following Tables 6 to 20 were obtained in the same manner as in the aforementioned Examples. Structures and physicochemical properties of the reference example compounds and Example compounds are respectively shown in the following Tables 2 to 5 and 6 to 20. In addition, the compounds whose chemical structures are shown in Tables 21 and 22 can be produced easily in almost the same manner as the

methods of the aforementioned Examples or production methods, or by applying thereto slight modifications obvious to those skilled in the art.

Abbreviations in the tables respectively indicate, Rex: reference example number ; Ex: Example number ; Co: compound number ; Str: structure ; Sal: salt (blank: free base or free acid ; Su: succinate ; HCl: hydrochloride) ; Sy: production method (each numeral shows the number of the aforementioned Example, indicating that said compound was produced by the same method of this aforementioned Example) and Rsy: reference example production method (each numeral shows the number of the aforementioned Reference Example, indicating that said compound was produced by the same method of this aforementioned Reference Example) ; Dat: physicochemical property ; Me: methyl ; Et: ethyl ; Pr: n-propyl ; iPr: isopropyl cPr: cyclopropyl ; Bu: n-butyl ; iBu: isobutyl ; cHex: cyclohexyl ; Ph: phenyl Bz: benzoyl ; Bn: benzyl ; Ac: acetyl Ms: methylsulfonyl ; Thie3: 3-thienyl ; Py2: 2-pyridyl ; Py3: 3-pyridyl ; Py4: 4-pyridyl ; Thia4: 4-tiazolyl ; Pip1: 1-piperidyl ; Pip2: 2-piperidyl ; Pip3: 3-piperidyl ; Pip4: 4-piperidyl ; Morp: morpholino ; Pipera: 1-piperazinyl ; Pim: 4-methyl-1-piperazinyl ; Im1: 1-imidazolyl ; Im2: 2-imidazolyl ; Fu3: 3-furyl ; Pyrr2: 2-pyrrolyl ; Pyrim2: 2-pyrimidinyl ; Pyrim5: 5-pyrimidinyl ; Pyra: 2-pyrazinyl ; Tet: 2H-tetrazol-5-yl ; Thiom: thiomorpholino ; Boc: tert-butoxycarbonyl ; and Pht: phthalimid-2-yl. In this case, the numeral before each substituent indicates its substituted position, and when two or more of $R^1$ or $R^2$ group are present, they are listed in the table together with their substituted positions.

For example, 4-OMe-5,6-$F_2$ indicates that methoxy is substituted at the 4-position, and F atom at the 5- and 6-positions.

**Table 2**

| Rex Rsy | Str | Dat | Rex Rsy | Str | Dat |
|---|---|---|---|---|---|
| 1 A1 | 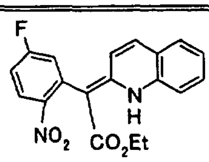 | F+:355 | 2 A1 | 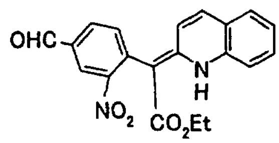 | F-:363 |
| 3 A1 | 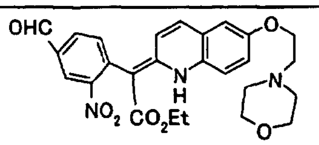 | F+:494 | 4 A1 | 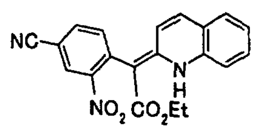 | F+:362 |
| 5 B1 | 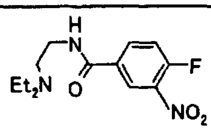 | F+:284 | 6 B1 | 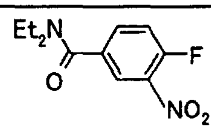 | F+:241 |

Table 3

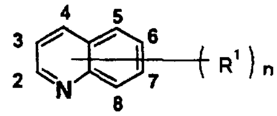

| Rex Rsy | $(R^1)n$ | Dat | Rex Rsy | $(R^1)n$ | Dat |
|---|---|---|---|---|---|
| 7 E2 | 6-$O(CH_2)_3CO_2Et$ | F+:260 | 8 E3 | 6-$OCH_2$-(1-Boc-Pip4) | F+:343 |
| 9 E2 | 6-$OCH_2CO_2Et$ | F+:232 | 10 E4 | 6-$O(CH_2)_3Br$ | N2:2.40(2H,qui),3.66(2H,t),4.24(2H,t),7.10(1H,d),7.33-7.39(2H,m),8.01(1H,d),8.04(1H,dd),8.77(1H,dd) |
| 11 I3 | 6-OMe-7-$(CH_2)_3OH$ | F+:218 | 12 E4 | 6-$O(CH_2)_4Br$ | N2:1.99-2.17(4H,m),3.53(2H,t),4.14(2H,t),7.10(1H,d),7.35-7.42(2H,m),7.97(1H,d),8.10(1H,dd),8.70(1H,dd) |
| 13 I4 | 6-$(CH_2)_3OH$-7-OMe | F+:218 | 14 E4 | 6-$O(CH_2)_5Br$ | N2:1.68(2H,qui),1.85-2.02(4H,m),3.46(2H,t),4.09(2H,t),7.05(1H,d),7.34(1H,dd),7.37(1H,dd),7.99(1H,d),8.07(1H,dd),8.76(1H,dd) |
| 15 L | 4-Cl-6-C≡$CCH_2OH$-7-OMe | F+:248 | 16 K | 6-$(CH_2)_3Br$-7-OMe | N2:2.23(2H,qui),2.95(2H,t),3.44(2H,t),3.98(3H,s),7.27(1H,t),7.42(1H,s),7.57(1H,s),8.04(1H,dd),8.79(1H,dd) |
| 17 R | 7-$(CH_2)_2CO_2Et$ | F+:230 | 18 K | 6-OMe-7-$(CH_2)_3Br$ | F+:280,282 |

**Table 4**

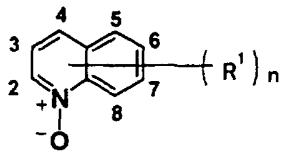

| Rex Rsy | $(R^1)n$ | Dat | Rex Rsy | $(R^1)n$ | Dat |
|---|---|---|---|---|---|
| 19 H1 | 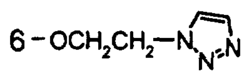 | F+:257 | 20 H1 | 4-$CH_2OBz$ | N2:5.77(2H,s), 8.60(1H,d), 8.85(1H,dd) |
| 21 H1 | 4-$(CH_2)_2CO_2H$ | F+:218 | 22 H1 | 4-$O(CH_2)_2Br$ | F+:268, 270 |
| 23 H1 | 6-$O(CH_2)_2OMe$ | F+:220 | 24 C | 6-$O(CH_2)_2NEt_2$ | F+:261 |
| 25 H1 | 6-$O(CH_2)_3CO_2Et$ | F+:276 | 26 H1 | 6-$O(CH_2)_2Br$-7-OMe | F+:298,300 |
| 27 H1 | 6-$OCH_2CO_2Et$ | F+:248 | 28 H1 | 6-$CH_2Br$ | F+:238, 240 |
| 29 H1 | 6-$OCH_2$(1-Boc-Pip4) | F+:359 | 30 H1 | 6-$O(CH_2)_3Br$ | F+:282, 284 |
| 31 H1 | 6-$(CH_2)_2CO_2H$ | F+:218 | 32 H1 | 6-$O(CH_2)_4Br$ | F+:296, 298 |
| 33 H1 | 6-$(CH_2)_3Br$-7-OMe | F:296, 298 | 34 H1 | 6-$O(CH_2)_5Br$ | F+:310, 312 |
| 35 H1 | 6-$CH_2OH$ | F+:176 | 36 H1 | 5-$O(CH_2)_2Br$ | F+:268,270 |
| 37 H1 | 5-$CH_2$-Pht-6-$O(CH_2)_2OMe$ | F+:379 | 38 H1 | 6-OBn | N1:5.26(2H,s), 8.43(1H,br d), 8.45(1H,d) |
| 39 H1 | 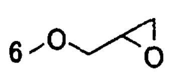 | F+:218 | 40 H1 | 6-$(CH_2)_3Br$ | N2:2.27(2H,qui),3.00(2H,t),3.42(2H,t),7.29(1H,dd),7.62(1H,dd),7.69(1H,s),8.58(1H,d),9.39(1H,d),9.56(1H,d) |
| 41 H1 | 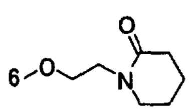 | F+:287 | 42 H1 | 7-$O(CH_2)_2Br$ | N1:3.90(2H,t),4.54(2H,t),7.35(1H,dd),7.42(1H,dd),7.90(1H,d),7.91(1H,s),8.04(1H,d),8.57(1H,dd) |
| 43 H1 | 4-$(CH_2)_2CO_2Et$ | F+:246 | 44 H3 | 6-CHO | N1:7.61(1H,dd),8.13-8.21(2H,m), 8.67(1H,d), 8.64-8.76 (2H,m), 10.20-10.23(1H,m) |

Table 5

| Rex Rsy | Str | Dat | Rex Rsy | Str | Dat | Rex Rsy | Str | Dat |
|---|---|---|---|---|---|---|---|---|
| **45** V | 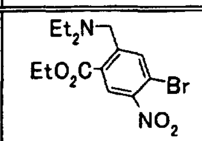 | F-:358, 360 | **46** E1 | 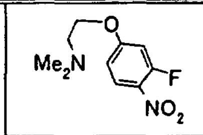 | F+:229 | **47** C | 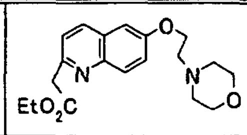 | F+:345 |
| **48** E1 | 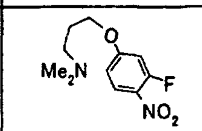 | F+:243 | **49** E1 | 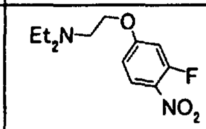 | F+:257 | **50** E1 | 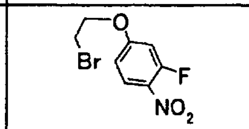 | E:263, 265 |
| **51** E2 | 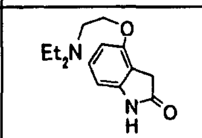 | F+:249 | **52** E2 | 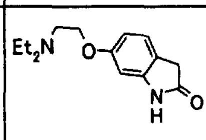 | F+:249 | **53** E3 | 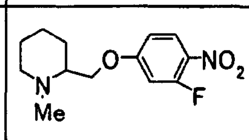 | F+:269 |
| **54** E3 | 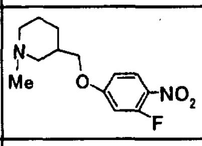 | F+:255 | **55** E4 | 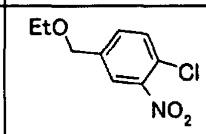 | F+:216 | **56** DD | 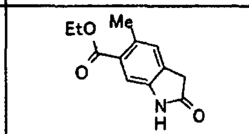 | F+:220 |
| **57** I2 | 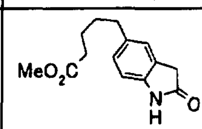 | F+:248 | **58** H2 | 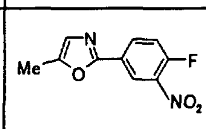 | F+:223 | **59** J2 | 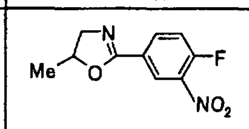 | F+:225 |
| **60** I2 | 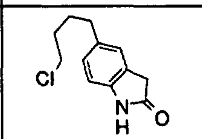 | F+:224 | **61** J2 | 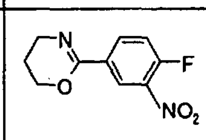 | F+:225 | **62** J2 | 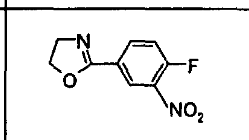 | F+:211 |
| **63** J2 | 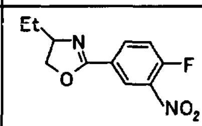 | F+:239 | **64** M1 | 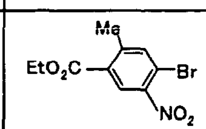 | F+:287 ,289 | **65** M2 | 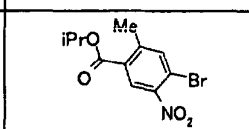 | F+:302 ,304 |
| **66** M2 | 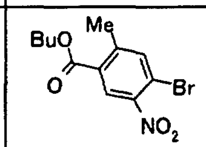 | F+:315 ,317 | **67** P | 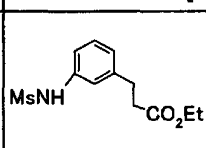 | F+:272 | **68** N | 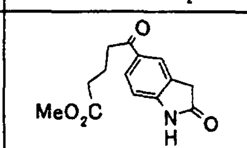 | F+:262 |
| **69** O | 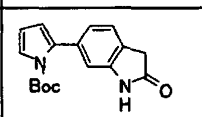 | F+:299 | **70** BB | 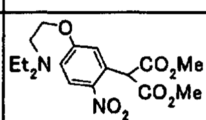 | F+:369 | **71** Q | 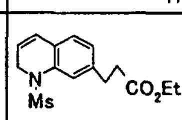 | N2:1.25(3H, t),2.69(3H,s) |
| **72** BB | 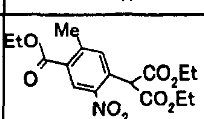 | F+:368 | **73** BB | 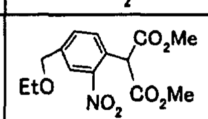 | F+:312 | **74** BB | 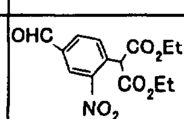 | N2:5.35(1H, s),10.10(1H, s) |
| **75** CC2 | 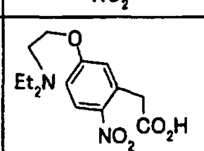 | F+:297 | **76** DD | 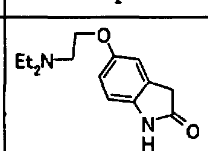 | F+:249 | **77** CC1 | 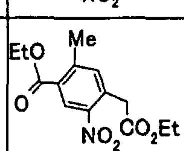 | N2:2.96(3H, s),4.03(2H,s ),7.23(1H,s), 8.69(1H,s) |
| **78** DD | 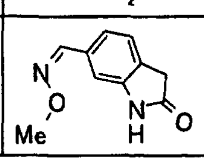 | F+:191 | **79** DD | 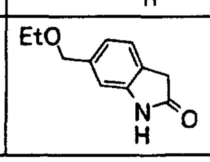 | F+:192 | **80** DD | 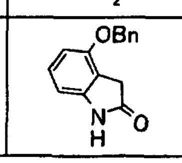 | F+:240 |

**Table 6**

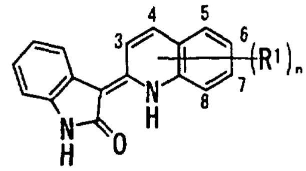 (I)

| Ex | $(R^1)n$ | Sal | Sy | Dat |
|---|---|---|---|---|
| **1** | 6— $OCH_2CH_2$— 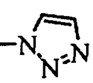 | | - | N1:4.49(2H,t), 4.84(2H,t), 7.76(1H,d), 8.23(1H,d), 10.53(1H,s), 14.43(1H,s) ; F+:372 |
| **2** | 7-$CO_2Et$ | | - | F+:333 |
| **3a** | 4-CHO | | - | N1:10.66(1H,s),12.60(1H,s),14.10(1H,s) |
| **3b** | 4-$CH(OEt)_2$ | | - | F+:363 |
| **4** | 6-$O(CH_2)_2$-Morp | | - | N1:2.51(4H,t), 2.78(2H,t), 3.59(4H,t), 4.16(2H,t), 10.53(1H,s), 14.46(1H,s) ; F+:390 |
| **5** | 6-$CH_2NH(CH_2)_2$-Morp | | - | N1: 3.55 (4H, t), 3.78 (2H, s), 10.56 (1H, s) ; F+: 403 |
| **6** | 6-$CH_2N(Me)(CH_2)_2OMe$ | | - | N1: 2.19 (2H, s), 2.25-2.60 (4H, m), 3.53 (2H, s), 10.57 (1H, s), 14.38 (1H, s) ; F+: 361 |
| **7** | 6 – 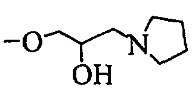 | | - | N1:1.65-1.71(4H,m),2.42-2.54(5H,m),2.64(1H,dd),3.90-4.00(2H,m),4.04-4.12(1H,m),4.93(1H,d),10.51(1H,s),14.47(1H,s) ; F+:404 |
| **8** | 6-$OCH_2CO$-Morp | | - | N1: 3.40-3.73 (8H, m), 4.92 (2H, s), 10.53 (1H, s), 14.46(1H, s) ; F: 403 |
| **9** | 6-$NHCOCH_2$-Morp | | - | F-:401 |
| **10** | 6-$NH_2$ | | - | N1:5.39(2H,s), 10.43(1H,s), 14.52(1H,s) ; F-:274 |
| **11** | 6 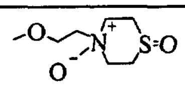 | | - | F+:438 |
| **12** | 6-CO-Pipera | HCl | - | F+:373 |
| **13** | 5-$CH_2NH_2$-6-$O(CH_2)_2OMe$ | HCl | - | N1:3.35(3H,s),3.72-3.76(2H,m),4.28-4.33(2H,m),4.34-4.41(2H,m),10.59(1H,s) ; F+:364 |
| **14** | 6-$CH_2OH$ | | - | N1:4.57(2H,s), 10.57(1H,s), 14.40(1H,s) ; F+:291 |
| **15** | 6-$OCH_2$(1-Me-Pip4) | | - | N1:2.50(3H,s),3.90(2H,d),10.51(1H,s),14.46(1H,s) ; F+:388 |
| **16** | 6-NHCONH-Py3 | | - | N1:10.55(1H,s), 14.42(1H,s) ; F-:394 |
| **31** | 6-$OCH_2CH_2Br$ | | 1 | F+:283, 285 |
| **32** | 2:1mixture of 7-Me and 5-Me | | 1 | N2:2.44(3H × 2/3,s), 2.57(3H × 1/3,s), 10.57(1H,s), 14.37(1H,s) ; F+:275 |
| **33** | 6-$OCH_2CH_2CH_2Br$ | | 1 | N1:2.30(2H,qui), 3.71(2H,t), 4.17(2H,t), 10.54(1H, s), 14.46(1H,s) |
| **34** | 6-$O(CH_2)_4Br$ | | 1 | F+:411, 413 |
| **35** | 6-$O(CH_2)_5Br$ | | 1 | F+:425, 427 |
| **36** | 6-OBz | HCl | 1 | N1:10.61(1H,s), 14.39(1H,s) ; F+:381 |

Table 7

| | | | | |
|---|---|---|---|---|
| 37 | 6-OMe | HCl | 1 | N1:3.85(3H,s), 10.52(1H,s), 14.47(1H,s) ; F+:291 |
| 38 | 4-Me | | 1 | N2:2.66(3H,s), 10.55(1H,s), 14.35(1H,s) ; F+:275 |
| 39 | 4-$CH_2CH_2CO_2Et$ | | 1 | N2:1.17(3H,s), 2.84(2H,t), 3.32(2H,t), 4.09(2H,q), 10.55(1H,s), 14.29(1H,s) ; F+:361 |
| 40 | 4-$CH_2OBz$ | | 1 | N1:5.84(2H,s), 10.60(1H,s), 14.16(1H,s) ; F:394 |
| 41 | 6-O$(CH_2)_2$-Pim | | 4 | N1:2.15(3H,s), 2.25-2.40(4H,m), 2.45-2.55(4H,m), 2.72(2H,t), 4.15(2H,t), 10.52(1H,s), 14.46(1H,s) ; F+:403 |
| 42 | 6-O$(CH_2)_3$-Morp | | 4 | N1:1.92(2H,qui), 2.35-2.41(4H,m), 2.45(2H,t), 3.58(4H,t), 4.09(2H,t), 10.51(1H,s), 14.47(1H,s) ; F+:404 |
| 43 | 6-$CH_2OBz$ | | 1 | N1:5.43(2H,s), 10.61(1H,s), 14.35(1H,s) ; F+:395 |
| 44 | 6-$OCH_2CH_2$-$NEt_2$ | Su | 1 | N1:1.02(6H,t), 2.39(4H,s), 2.65(4H,q), 2.91(2H,t), 4.13(2H,t), 10.53(1H,s), 14.46(1H,s) ; F+:376 |
| 45 | 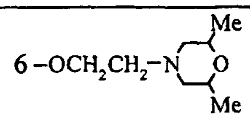 | | 4 | N1:1.05(6H,d), 1.74(2H,t), 2.71(2H,t), 2.84(2H,d), 3.53-3.60(2H,m), 4.16(2H,t), 10.51(1H,s), 14.46(1H,s) ; F+:418 |
| 46 | 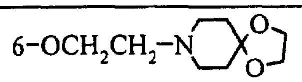 | | 4 | N1:1.62(4H,t), 2.57(4H,brt), 2.76(2H,t), 3.86(4H, s), 4.15(2H,t), 10.51(1H,s), 14.46(1H,s) ; F+:446 |
| 47 | 6-O$(CH_2)_4$-Morp | | 4 | N1:1.56-1.63(2H,m), 1.74-1.81(2H,m), 2.32-2.35(6H,m), 3.56(4H,t), 4.07(2H,t), 10.51(1H,s), 14.46(1H,s) ; F+:418 |
| 48 | 6-O$(CH_2)_5$-Morp | | 4 | N1:1.44-1.50(4H,m), 1.74-1.83(2H,m), 2.27-2.30(2H,m), 2.33(4H,brs), 3.56(4H,t), 4.05(2H,t), 10.51(1H,s), 14.46(1H,s) ; F+:445 |
| 49 | 6-$NO_2$ | | 1 | E+:306 |
| 50 | 8-Me | | 1 | E+:275 |
| 51 | 4-Cl | | 1 | -:295 |
| 52 | 6-OBn | | 1 | N1:5.19(2H,s), 10.53(1H,s), 14.45(1H,s) ; F+:367 |
| 53 | 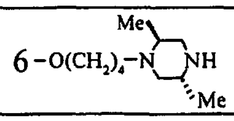 | Su | 4 | N1:1.00(3H,d), 1.05(3H,d), 10.52(1H,s), 14.46(1H,s) ; F+:445 |
| 54 | 6-$CH_2$-Morp | | 4 | N1:2.39(4H,brs), 3.54(2H,s), 3.59(4H,t), 10.57(1H,s), 14.38(1H,s) ; F+:360 |
| 55 | 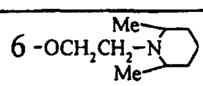 | | 4 | N1:1.10(6H,d), 10.52(1H,s), 14.47(1H,s) ; F+:416 |
| 56 | 6-Br | | 1 | N1:10.63(1H,s), 14.27(1H,s) ; F+:338, 340 |

**Table 8**

| | | | | |
|---|---|---|---|---|
| **57** | 6 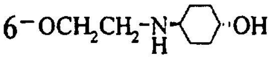 | | 4 | N1:10.51(1H,s), 14.46(1H,s) ; F+:418 |
| **58** | 6-O($CH_2$)$_2$OMe | | 19 | N1: 3.33 (3H, s), 3.65-3.74 (2H, m), 4.11-4.21 (2H, m), 10.53 (1H, s) ; F+: 335 |
| **59** | 6 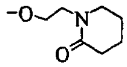 | | 19 | N1: 1.60-1.80 (4H, m), 2.17-2.27 (2H, m), 10.52 (1H, s), 14.45 (1H, s) ; F+: 402 |
| **60** | 6 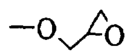 | | 19 | N1:2.76(1H,td),2.88(1H,t),3.38-3.43(1H,m),3.94(1H,td),4.44(1H,ddd),10.53(1H,s),14.46(1H,s) |
| **61** | 6-O($CH_2$)$_3$$CO_2$Et | | 1 | F+:391 |
| **62** | 6-O$CH_2$$CO_2$Et | | 1 | F+:363 |
| **63** | 5-$CH_2$Pht-6-O($CH_2$)$_2$OMe | | 1 | F+:494 |
| **64** | 6-O$CH_2$(1-Boc-Pip4) | | 1 | F+:474 |
| **65** | 6-$CO_2$H | | 1 | F+:305 |
| **66** | 6-($CH_2$)$_2$$CO_2$H | | 1 | N1: 2.61(2H,t),2.91(2H,t),10.55(2H,s),12.23 (1H,s),14.35(1H,s) ; F+:333 |
| **67** | 6-($CH_2$)$_3$Br-7-OMe | | 1 | F+:411, 413 |
| **68** | 6-CHO | | 1 | N1:6.91-7.06(3H,m),7.64(1H,d),7.68(1H,d),7.78(1H,d),8.03(1H,d),8.09(1H,d),8.28(1H,s),9.98(1H,s),10.70(1H,s),14.29(1H,s) |
| **69** | 4-($CH_2$)$_2$$CO_2$H | | 1 | N1: 2.61(2H,t),2.91(2H,t),10.55(2H,s),12.23 (1H,s),14.35(1H,s) ; F+:333 |
| **70** | 4-$CO_2$H | | 1 | F+:305 |
| **71** | 4-O($CH_2$)$_2$Br | | 1 | F:382, 384 |
| **72** | 5-$NO_2$ | | 1 | F-:304 |
| **73** | 6-O($CH_2$)$_2$Cl | | 1 | F+:339 |
| **74** | 6-OMe-7-($CH_2$)$_3$Br | | 2 | F+:411, 413 |
| **75** | 7-($CH_2$)$_3$Br | | 2 | F+:381, 383 |
| **76** | 7-($CH_2$)$_2$$CO_2$Et | | 2 | F+:361 |
| **77** | 6-OMe-7-($CH_2$)$_3$Morp | | 4 | F+:418 |
| **78** | 6-O($CH_2$)$_2$(4-OH-Pip1) | | 4 | N1:.1.35-1.44(2H,m),1.68-1.75(2H,m),2.15(2H,t),2.70(2H,t),2.78-2.83(2H,m),3.41-3.49(1H,m),4.13(2H,t),4.53(1H,d),10.51(1H,s),14.46(1H,s) ; F+:404 |
| **79** | 6-O($CH_2$)$_2$(4-$CO_2$Et-Pip1) | | 4 | F+:460 |
| **80** | 6 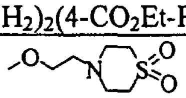 | | 4 | F+:438 |
| **81** | 6 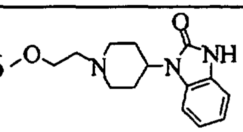 | | 4 | F+:520 |
| **82** | 6 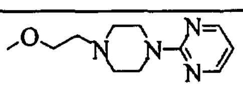 | | 4 | N1:6.62(1H,t),8.35(2H,d),10.52(1H,s),14.46(1H,s) ; F+:467 |
| **83** | 6 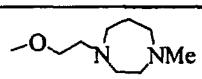 | | 4 | F+:417 |
| **84** | 6-O($CH_2$)$_2$(4-Pip1-Pip1) | | 4 | F+:471 |

Table 9

| | | | | |
|---|---|---|---|---|
| **85** | 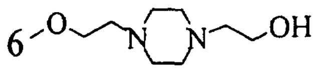 | | 4 | N1:2.37(2H,t),2.35-2.50(8H,m),2.72(2H,t),3.48(2H,q),4.14(2H,t),4.36(1H,t),10.52(1H,s),14.46(1H,s) ; F+:433 |
| **86** | 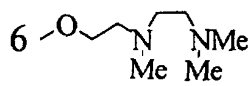 | | 4 | N1:2.14(6H,s),2.28(3H,s),2.34(2H,dd),2.48-2.53(2H,m),2.78(2H,t),4.13(2H,t),10.52(1H,s),14.46(1H,s) ; F+:405 |
| **87** | 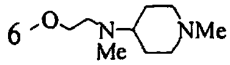 | HCl | 4 | F+:431 |
| **88** | 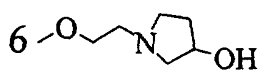 | | 4 | N1:1.5-1.6(1H, m), 1.9-2.1(1H, m), 2.4-2.5(1H, m), 2.6-2.8(4H, m), 4.1-4.3(3H, m), 4.6-4.7(1H, m), 10.51(1H, s), 14.46(1H, s) ; F+:390 |
| **89** | 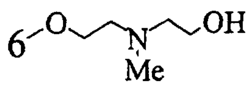 | | 4 | N1:2.30(3H, s), 2.5-2.6(2H, m), 2.79(2H, t), 3.4-3.6(2H, m), 4.1-4.2(2H, m), 4.37(1H, t), 10.52(1H, s), 14.47(1H, s) ; F-:376 |
| **90** | 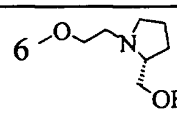 | | 4 | N1:1.5-1.9(4H, m), 2.3-2.4(1H, m), 2.6-2.7(1H, m), 3.1-3.5(4H, m), 4.13(2H, t), 4.39(1H, t), 10.52(1H, s), 14.47(1H, s) ; F+:404 |
| **91** | 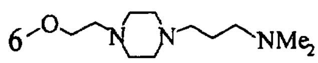 | | 4 | N1:1.4-1.6(2H, m), 2.11(6H, s), 2.2-2.5(12H, m), 2.72(2H, t), 4.15(2H, t), 10.51(1H, s), 14.46(1H, s) ; F+:474 |
| **92** | 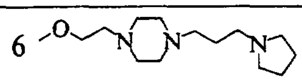 | | 4 | N1:1.5-1.7(6H, m), 2.2-2.5(12H, m), 2.72(2H, t), 4.15(2H, t), 10.51(1H, s), 14.46(1H, s) ; F+:500 |
| **93** | 6-O($CH_2$)$_2$(4-$CONH_2$-Pip1) | | 4 | F+:431 |
| **94** | 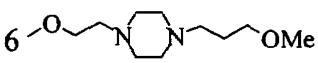 | | 4 | N1:1.63(2H,qui),2.29(2H,t),2.30-2.42(8H,m),2.72(2H,t),3.21(3H,s),3.32(2H,t),4.15(2H,t),10.52(1H,s),14.46(1H,s) ; F+:461 |
| **95** | 6-O($CH_2$)$_2$-Thiom | | 4 | F+:406 |
| **96** | 6-($CH_2$)$_3$-Morp | | 4 | N1:1.78(2H,qui),2.30(2H,t),2.32-2.38(4H,m),2.69(2H,t),3.58(2H,t),10.55(1H,s),14.38(1H,s) ; F+:383 |
| **97** | 6-($CH_2$)$_3$-Morp-7-OMe | | 4 | F+:418 |
| **98** | 6-($CH_2$)$_3$-Pim | HCl | 4 | N1:2.05-2.15(2H,m),2.76(2H,t),2.83(3H,s),3.12-3.85(10H,m),10.58(1H,s),14.34(1H,s) ; F+:401 |
| **99** | 6-($CH_2$)$_3$$NEt_2$ | | 4 | N1:0.93(6H,t),1.73(2H,qui),2.39(2H,t),2.45(4H,q),2.66(2H,t),10.55(1H,s),14.38(1H,s) ; F+:374 |
| **100** | 4-O($CH_2$)$_2$-Morp | | 4 | F-:388 |
| **101** | 7-($CH_2$)$_3$-Morp | | 4 | N1: 3.58(4H,t), 10.57(1H,s), 14.36(1H,s) ; F+:388 |
| **102** | 7-O($CH_2$)$_2$-Morp | | 4 | N1:2.48-2.52(4H,m),2.74(2H,t),3.60(4H,t),4.26(2H,t),10.55(1H,s),14.38(1H,s) ; F+:390 |
| **103** | 7-($CH_2$)$_3$$NEt_2$ | | 4 | N1: 0.94(4H,t), 10.55(1H,s), 14.37(1H,s) ; F+:374 |
| **104** | 7-($CH_2$)$_3$-Pim | | 4 | N1: 2.14(3H,s), 10.57(1H,s), 14.36(1H,s) ; F+:401 |

Table 10

| | | | | |
|---|---|---|---|---|
| **105** | 5-O$(CH_2)_2$-Morp | | 4 | N1:2.54(4H,t),2.83(2H,t),3.60(4H,t),4.27(2H,t),10.57(1H,s),14.34(1H,s) ; F+:390 |
| **106** | 6 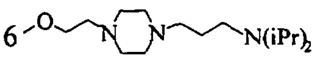 | | 4 | N1:0.94(12H,d),2.2-2.3(2H,m),2.71(2H,t),2.9-3.0(2H,m),4.15(2H,t),10.51(1H,s),14.46(1H,s) ; F+:516 |
| **107** | 6 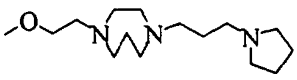 | | 4 | N1:1.5-1.7(8H, m), 2.3-2.8(16H, m), 2.89(2H, t), 4.12(2H, t), 10.51(1H, s), 14.46(1H, s) ; F+:514 |
| **108** | 6 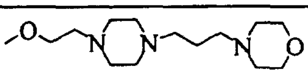 | | 4 | N1:1.55(2H,t),2.3-2.5(12H,m),2.72(2H,t),3.5-3.6(4H,m),4.15(2H,t),10.51(1H,s),14.46(1H,s) ; F+:516 |
| **109** | 6 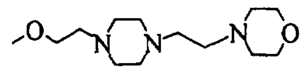 | | 4 | N1;2.3-2.5(12H,m),2.71(2H,t), 3.5-3.6(4H,m), 4.14(2H,t),10.51(1H,s),14.46(1H,s) ; F+:502 |
| **110** | 6-$(CH_2)_3$(4-OH-Pipl) | | 4 | F+:402 |
| **111** | 6 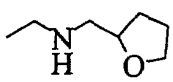 | | 5 | N1:1.49-1.57(1H,m),1.75-1.82(2H,m),1.86-1.94(1H,m),2.14(1H,brs),2.53-2.57(2H,m),3.60(1H,q),3.80(2H,s),3.89(1H,q),10.56(1H,s),14.39(1H,s) ; F-:372 |
| **112** | 6-$CH_2NH(CH_2)_2OMe$ | | 5 | N1:2.33(1H,s),2.66(2H,t),3.24(3H,s),3.42(2H,t),3.78(2H,s),10.57(1H,s),14.39(1H,s) ; F+:348 |
| **113** | 6 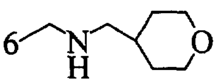 | HCl | 5 | N1:1.17-1.27(2H,m),1.68(2H,dd),1.95-2.05(1H,m),2.85(2H,s),3.28(2H,td),3.85(2H,dd),4.21(2H,s),10.63(1H,s),14.31(1H,s) ; F:387 |
| **114** | 6 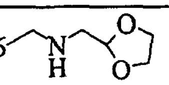 | | 5 | N1: 2.66 (2H, d), 4.91 (1H, t), 10.55 (1H, s), 14.38 (1H, s) ; F: 375 |
| **115** | 6 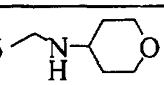 | | 5 | N1: 1.22-1.37 (2H, m), 1.75-1.86 (2H, m), 2.58-2.69 (1H, m), 10.57 (1H, s) ; F: 373 |
| **116** | 6 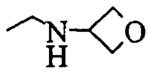 | | 5 | N1: 3.70 (2H, s), 3.90 (1H, qui), 10.57 (1H, s), 14.38 (1H, s) ; F: 345 |
| **117** | 6-$CH_2NH(CH_2)_3OMe$ | | 5 | N1: 1.70 (2H, qui), 3.82 (2H, s), 10.58 (1H, s), 14.38 (1H, s) ; F+: 362 |
| **118** | 6 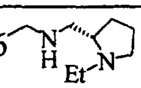 | | 5 | N1: 1.01 (3H, t), 3.79 (2H, s), 10.57 (1H, s), 14.39 (1H, s) ; F+: 401 |
| **119** | 6 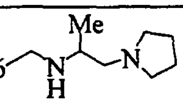 | | 5 | N1: 0.97 (3H, d), 1.64 (4H, br), 3.72 (1H, d), 3.87 (1H, d), 10.57 (1H, s), 14.39 (1H, s) ; F-: 399 |
| **120** | 6 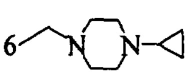 | | 5 | N1:0.24-0.29(2H,m),0.36-0.42(2H,m),1.56-1.62(1H,m),2.30-2.40(4H,m),2.50-2.58(4H,m),3.51(2H,s),10.57(1H,s),14.38(1H,s) ; F+:399 |
| **121** | 6 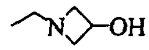 | | 5 | F:345 |
| **122** | 6 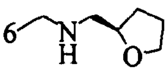 | | 5 | N1: 1.45-1.58 (1H, m), 1.70-2.00 (3H, m), 3.80 (2H, s), 10.57 (1H, s), 14.38 (1H, s) ; F+: 374 |

Table 11

| | | | | |
|---|---|---|---|---|
| **123** | 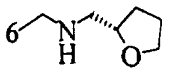 | | 5 | N1: 1.45-1.58 (1H, m), 1.70-2.00 (3H, m), 3.79 (2H, s), 10.57 (1H, s), 14.39 (1H, s) ; F+: 374 |
| **124** | 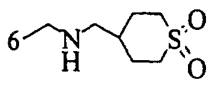 | | 5 | N1:1.53-1.63(2H,m),1.69-1.73(1H,m),2.09(2H,d), 2.42(2H,d),2.98-3.14(4H,m),3.75(2H,s),10.56(1H, s),14.38(1H,s) ; F:435 |
| **125** | 6-$CH_2NEt_2$ | | 5 | 1.00 (6H, br), 3.59 (2H, s), 10.58 (1H, s), 14.38 (1H, s) ; F: 345 |
| **126** | 6-$CH_2NHCH_2CO_2Et$ | | 5 | F+: 376 |
| **127** | 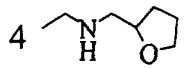 | | 5 | F:373 |
| **128** | 6-$CH_2$-Morp | | 5 | F+:360 |
| **129** | 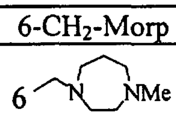 | | 6 | N1: 1.72 (2H, qui), 2.26 (3H, s), 3.66 (2H, s), 10.56 (1H, s), 14.38 (1H, s) ; F+: 387 |
| **130** | 6-$CH_2$-(4-$CONH_2$-Pip1) | | 6 | N1: 1.45-2.25 (7H, m), 3.51 (2H, s), 10.57 (1H, s), 14.38 (1H, s) ; F+: 401 |
| **131** | 6-$CH_2$-(4-Pyrim2-Pipera) | | 6 | N1: 2.40-2.54 (4H, m), 3.59 (2H, s), 3.72-3.82 (4H, m), 10.58 (1H, s), 14.39 (1H, s) ; F+: 437 |
| **132** | 6-$CH_2$-Pim | | 6 | N1: 2.21 (3H, s), 2.25-2.60 (4H, m), 3.53 (2H, s), 10.57 (1H, s), 14.38 (1H, s) ; F+: 373 |
| **133** | 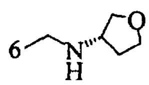 | HCl | 6 | N1:2.05-2.15(1H,m),2.15-2.27(1H,m),3.68(1H,q), 3.75-3.85(2H,m),3.89-3.96(2H,m),4.18(2H,dd),10. 64(1H,s),14.31(1H,s) ; F+:360 |
| **134** | 6-$CH_2$-(4-Et-Pipera) | | 6 | N1:0.98(3H,t),2.20-2.60(10H,m),3.53(2H,s),10.57( 1H,s),14.38(1H,s) ; F+:387 |
| **135** | 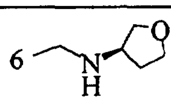 | HCl | 6 | N1:2.05-2.15(1H,m),2.15-2.27(1H,m),3.68(1H,q), 3.75-3.85(2H,m),3.89-3.96(2H,m),4.18(2H,dd),10. 64(1H,s),14.31(1H,s) ; F:359 |
| **136** | 6-$CH_2$-(4-Pr-Pipera) | | 6 | N1:0.84(3H,t),1.35-1.47(2H,m),3.52(2H,s),10.56(1 H,s),14.38(1H,s) ; F+:401 |
| **137** | 6-$CH_2$-(3,3-$F_2$-Pip1) | | 6 | N1:1.8-2.3(4H,m),2.7-3.3(1H,m),3.3-3.7(1H,m),3. 64(2H,brs),4.41(2H,brs),10.63(1H,s),14.29(1H,s) ; F+:394 |
| **138** | 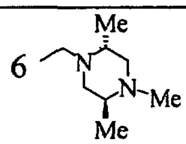 | | 6 | N1:0.80-0.95(3H,m),1.10(3H,d),2.17(3H,s),3.32(2 H,s),10.57(1H,s),14.38(1H,s) ; F+:401 |
| **139** | 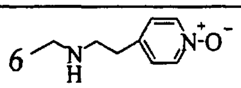 | | 6 | N1:2.7-2.8(4H,m),3.78(2H,s),7.27(2H,d),8.11(2H, d),10.57(1H,s),14.38(1H,s) ; F+:411 |
| **140** | 6-$CH_2$-(4-Ac-Pipera) | | 6 | N1: 1.98 (3H, s), 3.56 (2H, s), 10.58 (1H, s), 14.38 (1H, s) ; F+: 401 |
| **141** | 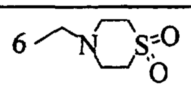 | | 6 | N1: 2.83-3.03 (4H, m), 3.05-3.20 (4H, m), 3.74 (2H, s), 10.58 (1H, s), 14.36 (1H, s) ; F+: 408 |
| **142** | 6-$CH_2$-(4-cHex-Pipera) | | 6 | N1: 0.97-1.27 (6H, m), 3.51 (2H, s), 10.57 (1H, s), 14.38 (1H, s) ; F+: 441 |

**Table 12**

| | | | | |
|---|---|---|---|---|
| **143** | 6 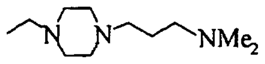 | | 6 | N1: 2.09 (6H, s), 3.51 (2H, s), 10.57 (1H, s), 14.38 (1H, s) ; F-: 442 |
| **144** | 6-$(CH_2)_2$CONHOCH$_2$-cPr | | 8 | F+:402 |
| **145** | 6-CONH$(CH_2)_2$-Morp | | 8 | F+:417 |
| **146** | 6-CONHCH$_2$CO$_2$Et | | 8 | F-:388 |
| **147** | 6-CONH$(CH_2)_2$-Py4 | | 8 | F+:409 |
| **148** | 6-CO-Pim | | 8 | N1:2.21(3H,s),2.30-2.40(4H,m),3.36-3.75(4H,m),10.63(1H,s),14.32(1H,s) ; F+:387 |
| **149** | 6-CONHMe | | 8 | N1:2.81(3H,d),8.51(1H,q),10.64(1H,s),14.31(1H,s) ; F+:318 |
| **150** | 6 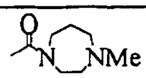 | | 8 | N1:2.25(3Hx1/2,s),2.29(3Hx1/2,s),10.62(1H,s),14.32(1H,s) ; F+:401 |
| **151** | 6-CON(Me)$(CH_2)_2$NEt$_2$ | | 8 | N1:0.70-1.10(6H,m),2.20-2.70(6H,m),3.31(3H,s),3.28-3.56(2H,m),10.62(1H,s),14.32(1H,s) |
| **152** | 6-CO(4-cPr-Pipera) | | 8 | N1:0.32-0.36(1H,m),0.42-0.46(1H,m),1.64-1.70(1H,m),2.52-2.62(4H,m),3.30-3.65(4H,m),10.63(1H,s),14.32(1H,s) ; F+:413 |
| **153** | 6-CO(4-Et-Pipera) | | 8 | N1:1.01(3H,t),2.36(2H,q),2.30-2.50(4H,m),3.30-3.70(4H,m),10.63(1H,s),14.32(1H,s) ; F+:401 |
| **154** | 6-CON(Me)OMe | | 8 | N1:3.31(3H,s),3.60(3H,s),10.64(1H,s),14.31(1H,s) ; F+:348 |
| **155** | 6-CO(4-NMe$_2$-Pip1) | | 8 | N1:1.3-1.5(2H,m),1.6-2.0(2H,m),2.21(6H,s),2.3-2.5(1H,m),3.6-3.9(1H,m),4.2-4.6(1H,m),10.63(1H,s),14.32(1H,s) ; F-:413 |
| **156** | 6 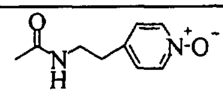 | | 8 | N1:2.87(2H,t),3.55(2H,q),7.29(2H,d),8.12(2H,d),8.62(1H,t),10.65(1H,s),14.30(1H,s) ; F+:425 |
| **157** | 6-CO(4-Boc-Pipera) | HCl | 8 | F+:473 |
| **158** | 6-CO(4-CO$_2$Et-Pip1) | | 8 | F+:444 |
| **159** | 4-CONH$(CH_2)_2$-Morp | | 8 | N1:2.56(2H,t),3.30(4H,s),3.49(2H,q),3.64(4H,t),8.92(1H,t),10.66(1H,s),14.21(1H,s) ; F+:417 |
| **160** | 4-CONH$(CH_2)_2$NEt$_2$ | | 8 | N1: 1.02 (6H, t), 2.58 (4H, q), 2.65 (2H, t), 8.88 (1H, t), 10.66 (1H, s), 14.22 (1H, s) ; F+: 403 |
| **161** | 4 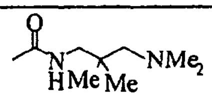 | | 8 | F+: 417 |
| **162** | 4-CON(Me)$(CH_2)_2$NEt$_2$ | HCl | 8 | F+:417 |
| **163** | 4-CONH$(CH_2)_3$-Morp | | 8 | N1:1.77(2H,qui),2.36-2.43(6H,m),3.39(2H,q),3.58(4H,t),8.99(1H,t),10.66(1H,s),14.23(1H,s) ; F+:431 |
| **164** | 4 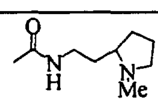 | | 8 | N1:1.42-1.77 (4H, m), 2.27 (3H, s), 9.00 (1H, br), 10.67 (1H, s), 14.21 (1H, s) ; F+: 415 |
| **165** | 4-CONH(4-NH$_2$-cHex) | | 8 | F+: 401 |
| **166** | 4-CO(4-Pip1-Pip1) | HCl | 8 | F+:455 |
| **167** | 4-CONH(3-NH$_2$-cHex) | | 8 | F+: 401 |

Table 13

| | | | | |
|---|---|---|---|---|
| **168** | 4 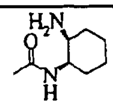 | | 8 | F: 400 |
| **169** | 4-CONHCH$_2$-Pyra | | 8 | N1:4.7-4.8(2H,m),8.61(1H,s),8.63(1H,s),8.79(1H,s),9.54-9.70(1H,m),10.67(1H,s),14.20(1H,s) ; F+:396 |
| **170** | 4-CONH-Pyrim5 | | 8 | N1:9.02(1H,s),9.20(2H,s),10.70(1H,s),11.33(1H,s),14.29(1H,s) ; F+:382 |
| **171** | 7-CONH(CH$_2$)$_2$-Morp | | 8 | F+: 417 |
| **172** | 7-CON(Me)(CH$_2$)$_2$-NEt$_2$ | | 8 | F+: 417 |
| **173** | 7-CO-Pim | | 8 | F+: 387 |
| **174** | 6 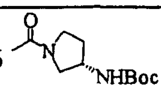 | | 8 | N1:1.23(9Hx1/2,s),1.34(9Hx1/2,s),7.22(1H,brs),10.63(1H,s),14.32(1H,s) ; F+:473 |
| **175** | 4 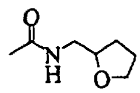 | | 8 | N1:1.63-1.71(1H,m),1.81-1.94(2H,m),1.95-2.05(1H,m),3.44(2H,td),3.69(1H,q),3.83(1H,q),4.07(1H,qui),9.07(1H,t),10.65(1H,s),14.21(1H,s) ; F+:388 |
| **176** | 6 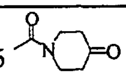 | | 8 | N1:2.45-2.52(4H,m),3.32(2H,s),3.65-3.90(4H,m),10.63(1H,s),14.32(1H,s) ; F-:384 |
| **177** | 5-NH$_2$ | | 10 | F:275 |
| **178** | 6-OH | | 10 | F:276 |
| **179** | 6-O(CH$_2$)$_2$(4-CO$_2$H-Pip1) | HCl | 23 | F+:432 |
| **180** | 6-O(CH$_2$)$_3$CO$_2$H | | 23 | F+:363 |
| **181** | 6-OCH$_2$CO$_2$H | | 23 | F-:333 |
| **182** | 6-CONHCH$_2$CO$_2$H | | 23 | F-:360 |
| **183** | 7-(CH$_2$)$_2$CO$_2$H | | 23 | F+: 333 |
| **184** | 6-OCH$_2$-Pip4 | | 12 | F+:374 |
| **185** | 6 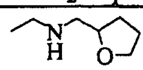 | | 12 | N1:3.87(2H,s), 10.58(1H,s), 14.42(1H,s) |
| **186** | 6 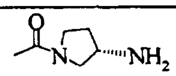 | HCl | 12 | F+:373 |
| **187** | 4-CH$_2$OH | | 14 | F+:291 |
| **188** | 5-NHCONH-CH$_2$CO$_2$Bu | | 16 | F+:433 |
| **366** | 6-CH$_2$NHCH$_2$(2-NH$_2$-Py4) | | 5 | N1:2.75(1H.brs),3.57(2H,s),3.75(2H,s),5.79(2H,s),6.46(1H,s),6.49(1H,d),7.82(1H,d),10.57(1H,s),14.39(1H,s) ; F+:396 |
| **367** | 6 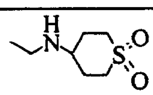 | | 5 | N1:3.88(2H,brs),10.59(1H,s),14.36(1H,s) ; F-:420 |
| **368** | 6-CO(4-Pim-Pip1) | | 6 | N1:2.14(3H,s),10.63(1H,s),14.32(1H,s) ; F+:470 |
| **369** | 6 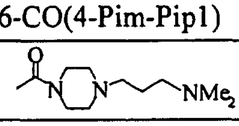 | | 8 | N1:1.55(2H,qui),2.11(6H,s),10.63(1H,s),14.32(1H,s) ; F+:458 |
| **370** | 6-CO(4-CO$_2$H-Pip1) | | 23 | N1:10.62(1H,s),12.30(1H,brs),14.30(1H,s) ; F+:416 |
| **371** | 6-CO(4-CO$_2$Et-Pip1) | | 8 | F+:444 |

Table 14

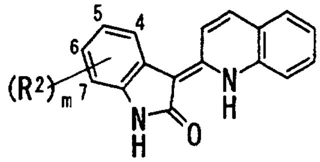

(I)

| Ex | $(R^2)m$ | Sy | Dat | Ex | $(R^2)m$ | Sy | Dat |
|---|---|---|---|---|---|---|---|
| **17** | 6-COOEt | - | F+:333 | **18** | $5\text{-NMe}_2$ | - | F+:304 |
| **19** | 6-OMe | - | F-:279 | **20** | 6-C(Me)=N-OMe | - | F+:332 |
| **21** | 6-Ac | - | F+:303 | **22** | 5-COOH | - | F+:305 |
| **23** | $5\text{-(CH}_2)_3\text{COOH}$ | - | F+:347 | **24** | 5-OH | - | F+:277 |
| **25** | 6-COOH | - | F-:303 | **26** | 6-Pyrr2 | - | F+:326 |
| **27** | 5-(2-Me-Thia4) | - | F+:358 | **28** | $5\text{-CO(CH}_2)_3\text{OH}$ | - | F+:347 |
| **189** | $6\text{-CONEt}_2$ | 17 | F+:360 | **190** | 6-CONH(CH2)2NEt2 | 17 | F+:403 |
| **191** | 6-CN | 17 | F+:286 | **192** | $6\text{-CH}_2\text{NEt}_2$ | 17 | F+:346 |
| **193** | 6-CHO | 17 | F+:289 | **194** | $6\text{-COO(CH}_2)_2\text{NMe}_2$ | 17 | F+:404 |
| **195** | 6-COOBn | 17 | F+:395 | **196** | $5\text{-OCH}_2\text{COOEt}$ | 17 | F+:363 |
| **197** | $6\text{-CONH}_2$ | 17 | F+:304 | **198** | $5\text{-Cl-6-CF}_3$ | 17 | F+:363 |
| **199** | 6-CONHEt | 17 | F+:332 | **200** | 5-Cl-6-COOEt | 17 | F+:367 |
| **201** | 5-Me | 17 | F:274 | **202** | $5\text{-OCH}_2\text{Py2}$ | 17 | F+:368 |
| **203** | $6\text{-CF}_3$ | 17 | F:328 | **204** | 5-F-6-COOEt | 17 | F+:351 |
| **205** | 6-Br | 17 | F:338, 340 | **206** | 6-COPr | 17 | F+:331 |
| **207** | 5-OMe | 17 | F-:289 | **208** | 6-Thia4 | 17 | F+:344 |
| **209** | 6-F | 17 | F-:277 | **210** | $5\text{-O(CH}_2)_2\text{NMe}_2$ | 17 | F+:348 |
| **211** | 7-F | 17 | F:278 | **212** | $5\text{-O(CH}_2)_3\text{NMe}_2$ | 17 | F+:362 |
| **213** | 5-F | 17 | F-:277 | **214** | $5\text{-OCH}_2\text{(1-Me-Pip2)}$ | 17 | F+:388 |
| **215** | $6\text{-SO}_2\text{Me}$ | 17 | F+:339 | **216** | 5-O(1-Me-Pip3) | 17 | F+:374 |
| **217** | 6-Cl | 17 | F:294 | **218** | $6\text{-CONHOCH}_2\text{cPr}$ | 17 | F+:374 |
| **219** | 4-Cl | 17 | F-:293 | **220** | 5-OMe-6-COOEt | 17 | F+:363 |
| **221** | 5-OBn | 17 | F+:367 | **222** | 5-Me-6-COOEt | 17 | F+:347 |
| **223** | $5\text{-CF}_3$ | 17 | F-:327 | **224** | $5\text{-CH}_2\text{NEt}_2\text{-6-COOEt}$ | 17 | F+:418 |
| **225** | 6 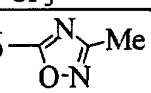 | 17 | F+:342 | **226** | 6 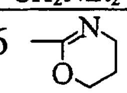 | 17 | F+:344 |
| **227** | 6 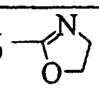 | 17 | F+:330 | **228** | 6 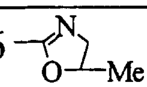 | 17 | F+:344 |
| **229** | 6 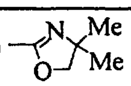 | 17 | F+:358 | **230** | 6 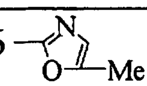 | 17 | F+:342 |
| **231** | 6-CH=N-OMe | 17 | F+:318 | **232** | $6\text{-CH}_2\text{CN}$ | 17 | F-:298 |
| **233** | $5\text{-O(CH}_2)_2\text{Br}$ | 17 | F+:383, 385 | **234** | 5-Morp | 17 | F+:346 |
| **235** | 5-Im1 | 18 | F+:327 | **236** | $5\text{-NH(CH}_2)_2\text{NEt}_2$ | 18 | F+:375 |
| **237** | 5-NHBn | 18 | F-:364 | **238** | $5\text{-COCH}_2\text{Morp}$ | 19 | F+:338 |
| **239** | 5-COOEt | 19 | F+:333 | **240** | 5-COMe | 19 | F+:303 |

Table 15

| | | | | | | | |
|---|---|---|---|---|---|---|---|
| **241** | 6-Me | 19 | F-:273 | **242** | 6-$F_2$ | 19 | F-:295 |
| **243** | 5-CN | 19 | E:285 | **244** | (Thie3) | 19 | F+:343 |
| **245** | 5-$(CH_2)_2$-Morp | 19 | F+:374 | **246** | $SO_2NH_2$ | 19 | F+:340 |
| **247** | 6-(2-OMe-Ph) | 19 | F:366 | **248** | $O(CH_2)_2NEt_2$ | 19 | F+:376 |
| **249** | 4-$O(CH_2)_2NEt_2$ | 19 | F+:376 | **250** | $O(CH_2)_2NEt_2$ | 19 | F+:376 |
| **251** | 5-$(CH_2)_3CO_2Et$ | 19 | F+:375 | **252** | 5-$CONH(CH_2)_2NEt_2$ | 19 | F-:401 |
| **253** | 5-$(CH_2)_4CO_2Me$ | 19 | F+:375 | **254** | 5-$CO(CH_2)_2CO_2Et$ | 19 | F+:389 |
| **255** | 6-Fu3 | 19 | F+:327 | **256** | $CO(CH_2)_3CO_2Me$ | 19 | F-:387 |
| **257** | 6-(1-Boc-Pyrr2) | 19 | F+:426 | **258** | 5-NHAc-6-Cl | 19 | F-:350 |
| **259** | 6-$CH_2OEt$ | 19 | F+:319 | **260** | 4-OBn | 19 | F+:367 |
| **261** | 5-$COCH_2Cl$ | 19 | F+:337 | **262** | 5-$CO(CH_2)_2Cl$ | 19 | F+:351 |
| **263** | 5-$CO(CH_2)_3Cl$ | 19 | F+:365 | **264** | 5-$(CH_2)_4Cl$ | 19 | F+:351 |
| **265** | 6-OCOMe | 19 | F+:317 | **266** | 7-OCOMe | 19 | F+:317 |
| **267** | 5-$COCH_2$-Im1 | 4 | F+:369 | **268** | 5-$COCH_2NMe_2$ | 4 | F+:344 |
| **269** | 5-$COCH_2$-Thiom | 4 | F+:404 | **270** | 5-$COCH_2$(4-OH-Pip1) | 4 | F+:402 |
| **271** | 5-$CO(CH_2)_2$-Morp | 4 | F+:402 | **272** | 5-$COCH_2$(3-OH-Pip1) | 4 | F+:402 |
| **273** | 5-$CO(CH_2)_2NMe_2$ | 4 | F+:360 | **274** | 5-$CO(CH_2)_2$-Im1 | 4 | F:383 |
| **275** | 5-$(CH_2)_4$-Im1 | 4 | F-:381 | **276** | 5-$CO(CH_2)_2N(Me)Bn$ | 4 | F:436 |
| **277** | 5-$COCH_2NEt_2$ | 4 | F+:374 | **278** | 6-$CONH(CH_2)_2OAc$ | 8 | F+:390 |
| **279** | 5-$CO(CH_2)_3COOH$ | 23 | F+:375 | **280** | 5-$OCH_2COOH$ | 23 | F+:335 |
| **281** | 4-OH | 24 | E:276 | **282** | 6-OH | 23 | F+:277 |
| **372** | 6-$(CH_2)_3NMe_2$ | 10 | F+:346 | **373** | 6-CH=N-$O(CH_2)_2NMe_2$ | 17 | F+:375 |

**Table 16**

| Ex | $(R^2)$m | Sal | Sy | Dat |
|---|---|---|---|---|
| 283 | 5-Cl | | 1 | N2:10.72(1H,s), 14.51(1H,s) ; F+:295 |
| 284 | 5-$NO_2$ | HCl | 1 | N1:11.30(1H,s), 14.45(1H,s) |
| 285 | 5-Br | HCl | 1 | N1:10.75(1H,s), 14.51(1H,s) ; F+:339, 401 |
| 286 | 5-$O(CH_2)_2NEt_2$ | | 19 | N1:1.01(6H,brs), 2.60(4H,brs), 2.80(2H,brs), 4.07(2H, brs), 10.39(1H,s), 14.46(1H,s) ; F+:376 |
| 287 | 6-(2-Me-Thia4) | | 17 | N1:2.72(3H,s), 10.69(1H,s), 14.33(1H, s) |
| 288 | 6 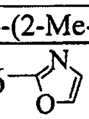 | | 17 | N1:7.32(1H,s),8.14(1H,s)10.83(1H,s),14.51(1H,s) ; F+:328 |
| 289 | 6 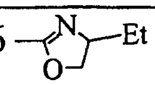 | | 17 | N1:0.94(3H,t),1.45-1.67(2H.m),3.99(1H,t),4.10-4.20(1 H,m),4.45(1H,t),10.78(1H,s),14.53(1H,s) ; F+:358 |
| 290 | 6-CH=N-OBn | | 29 | N1:5.15(2H, s),8.27(1H,s),10.71(1H,s),14.44(1H, s) ; F+:394 |
| 291 | 6-CH=N-$OCH_2$Py4 | | 29 | N1:5.47(2H,s),10.75(1H,s),14.44(1H,s) ; F+:395 |
| 292 | 5-COOAc | | 19 | N1:2.41(3H,s),10.97(1H,s),14.41(1H,s) |

Table 17

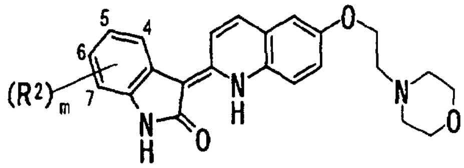

(I)

| Ex | $(R^2)m$ | Sal | Sy | Dat |
|---|---|---|---|---|
| 29 | 6-CH=N-OEt | HCl | - | N1:1.25(3H, t), 4.13(2H, q), 8.17(1H, s), 10.68(1H, s), 14.53(1H, s) ; F+:461 |
| 293 | 5-Cl | | 4 | N1:2.48-2.52(4H,m), 2.74(2H,t), 3.59(4H,t), 4.18(2H,t), 10.66(1H,s), 14.61(1H,s) ; F+:424 |
| 294 | 6-COOEt | | 17 | N1:1.33(3H,t), 4.29(2H,q), 10.76(1H, s), 14.76(1H, s) ; F+:462 |
| 295 | 6-CN | | 17 | N1:2.75(2H, m), 3.2-3.3(4H, m), 4.20(2H, t), 10.90(1H, s), 14.83(1H, s) ; F+:415 |
| 296 | $6-CF_3$ | | 17 | N1:2.7-2.8(2H, m), 3.5-3.6(4H, m), 4.1-4.2(2H, m), 10.85(1H, s), 14.72(1H, s) ; F+:458 |
| 297 | 6 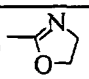 | | 17 | N1:3.93(2H,t), 4.34(2H,t), 10.72(1H, s), 14.64(1H, s) ; F+:459 |
| 298 | 5-Cl-6-COOEt | | 17 | N1:1.32(3H,t), 4.29(2H,q), 10.83(1H, s), 14.79(1H, s) ; F+:496 |
| 299 | 5-F-6-COOEt | | 17 | N1:1.31(3H,t), 4.28(2H,q), 10.73(1H, s), 14.84(1H, s) ; F+:480 |
| 300 | 5-OMe-6-COOEt | | 17 | F+:492 |
| 301 | 5-Me-6-COOEt | | 17 | N1:1.32(3H,t), 2.61(3H,s), 4.26(2H,q), 10.59(1H, s), 14.70(1H, s) ; F+:476 |
| 302 | 5-Me-6-COOMe | | 17 | N1:2.61(3H,s), 3.79(3H,s), 10.63(1H, s), 14.71(1H, s) ; F+:462 |
| 303 | 5-Me-6-COOiPr | | 17 | F+:490 |
| 304 | 5-Me-6-COOPr | | 17 | N1:0.99(3H,t), 1.67-1.78(2H,m), 2.62(3H,s), 10.59(1H, s), 14.71(1H, s) ; F+:490 |
| 305 | 5-Me-6-COOBu | | 17 | F+:504 |
| 306 | 5-Me-6-(CH=N-OMe) | | 17 | N1:2.42(3H, s), 3.82(3H, s), 8.37(1H, s), 10.50(1H, s), 14.50(1H, s) ; F+:461 |
| 307 | 6-CH=N-OMe | | 29 | N1:2.73(2H, t), 3.87(3H, s), 4.17(2H, t), 8.17(1H, s), 10.67(1H, s), 14.54(1H, s) ; F+:447 |
| 308 | 6-CH=N-OiBu | HCl | 29 | F+:489 |
| 309 | 6-CH=N-OBn | HCl | 29 | N1:5.15(2H, s), 8.26(1H, s), 10.68(1H, s), 14.54(1H, s) ; F+:523 |
| 310 | 6-F | | 4 | N1:2.73(2H, t), 3.5-3.6(4H, m), 4.16(2H, t), 10.66(1H, s), 14.32(1H, s) ; F+:408 |
| 311 | 5-F | | 4 | N1:2.7-2.8(2H, m), 3.5-3.6(4H, m), 4.18(2H, t), 10.53(1H, s), 14.62(1H, s) ; F+:408 |
| 312 | 6-Cl | | 4 | N1:2.74(2H, t), 3.2-3.4(4H, m), 3.5-3.6(4H, m), 4.18(2H, t), 10.67(1H, s), 14.44(1H, s) ; F+:424 |
| 313 | 6-COOH | | 23 | F+:434 |

Table 18

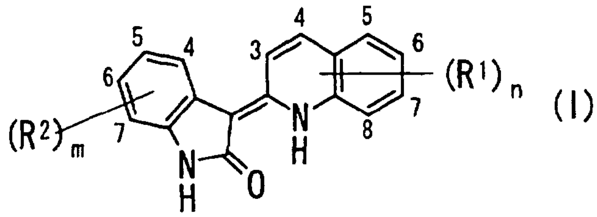

| Ex | $(R^2)m$ | $(R^1)n$ | Sal | Sy | Dat |
|---|---|---|---|---|---|
| 314 | 5-Me-6-$CO_2$Et | 6-O$(CH_2)_2$Br | | 19 | F+:469, 471 |
| 315 | 6-F | 6-$(CH_2)_3$Br | | 19 | F:399,401 |
| 316 | 6-F | 6-CHO | | 19 | N1:9.97(1H, s), 10.83(1H, s), 14.12(1H, s) |
| 317 | 5-Me-6-$CO_2$Et | 6-CHO | | 19 | F+:375 |
| 318 | 5-Me-6-$CO_2$Et | 6-$(CH_2)_3$Br | | 19 | F+:467, 469 |
| 319 | 6-CH=NOMe | 6-CHO | | 19 | F+:346 |
| 320 | 6-CH=NOMe | 6-$(CH_2)_3$Br | | 19 | F+:438, 440 |
| 321 | 5-Me-6-$CO_2$Et | 6-$CO_2$H | | 19 | N1:1.32(3H, t), 2.60(3H, s), 4.26(2H, q), 10.72(1H, s), 14.54(1H, s) ; F+:391 |
| 322 | 5-Cl | 6-O$(CH_2)_2$Br | | 1 | F:418 |
| 323 | 6-F | 6-O$(CH_2)_2$Br | | 1 | F:400, 402 |
| 324 | 5-F | 6-O$(CH_2)_2$Br | | 1 | F:400, 402 |
| 325 | 6-Cl | 6-O$(CH_2)_2$Br | | 1 | F:416, 418 |
| 326 | 5-$NO_2$ | 6-O$(CH_2)_2$Br | | 1 | E:427, 429 |
| 327 | 5-CN | 6-O$(CH_2)_2$Br | | 1 | F:407, 409 |
| 328 | 5,6-F2 | 6-O$(CH_2)_2$Br | | 1 | F+:419, 421 |
| 329 | 5-F | 6-$(CH_2)_2CO_2$H | | 1 | F+:351 |
| 330 | 6-F | 6-O$(CH_2)_2$Cl | | 1 | N1:4.00(2H, t), 4.25(2H, t),10.68(1H, s), 14.42(1H, s) |
| 331 | 6-Cl | 6-O$(CH_2)_2$Cl | | 1 | F-:355 |
| 332 | 5-Me-6-$CO_2$Et | 6-O$(CH_2)_2$Pim | 2HCl | 4 | N1:1.32(3H,t), 2.61(3H,s), 4.26(2H,q), 10.62(1H,s), 14.69(1H,s) ; F+:489 |
| 333 | 6-F | 6-O$(CH_2)_2$NEt$_2$ | | 4 | N1:1.01(6H, t), 2.59(4H, q), 2.7-2.8(2H, m), 4.09(2H, t), 10.65(1H, s), 14.32(1H, s) ; F+:394 |
| 334 | 6-F | 6-O$(CH_2)_2$Pim | | 4 | N1:2.17(3H, s), 2.3-2.5(4H, m), 2.73(2H, t), 4.15(2H, t), 10.65(1H, s), 14.32(1H, s) ; F+:421 |
| 335 | 5-F | 6-O$(CH_2)_2$NEt$_2$ | | 4 | N1:0.99(6H, t), 2.57(4H, q), 2.82(2H, t), 4.11(2H, t), 10.54(1H, s), 14.63(1H, s) ; F+:394 |
| 336 | 5-F | 6-O$(CH_2)_2$Pim | | 4 | N1:2.15(3H, s), 2.3-2.4(2H, m), 2.73(2H, t), 4.15(2H, t), 10.54(1H, s), 14.62(1H, s) ; F+:421 |
| 337 | 6-Cl | 6-O$(CH_2)_2$NEt$_2$ | | 4 | N1:1.00(6H, t), 2.5-2.7(4H, m), 2.8-2.9(2H, m), 4.11(2H, t), 10.67(1H, s), 14.45(1H, s) ; F+:410 |

Table 19

| | | | | | |
|---|---|---|---|---|---|
| **338** | 6-Cl | 6-O$(CH_2)_2$Pim | | 4 | N1:2.17(3H, s), 2.3-2.5(4H, m), 2.73(2H, t), 4.17(2H, t), 10.68(1H, s), 14.44(1H, s) ; F+:437 |
| **339** | 5-$NO_2$ | 6-O$(CH_2)_2$Morp | | 4 | F+:435 |
| **340** | 5-CN | 6-O$(CH_2)_2$Morp | | 4 | F+:415 |
| **341** | 5,6-$F_2$ | 6-O$(CH_2)_2$Morp | | 4 | N1:2.73(2H,t), 3.59(4H,t), 4.17(2H,t), 10.63(1H,s), 14.50(1H,s) : F+:426 |
| **342** | 5-Me-6-$CO_2$Et | 6-O$(CH_2)_2$$NEt_2$ | HCl | 4 | N1:1.27(6H,t), 1.32(3H, t), 2.61 (3H,s), 4.26(2H,q), 10.62(1H,s), 14.70(1H,s) ; F+:462 |
| **343** | 6-F | 6-$(CH_2)_3$$NEt_2$ | | 4 | N1:0.93(6H, t), 1.6-1.8(2H, m), 2.3-2.5(6H, m), 2.67(2H, t), 10.69(1H, s), 14.24(1H, s) ; F+:392 |
| **344** | 6-F | 6-$(CH_2)_3$Morp | | 4 | N1:1.7-1.9(2H, m), 2.2-2.4(6H, m), 2.6-2.7(2H, m), 3.4-3.6(2H, m), 10.69(1H, s), 14.23(1H, s) ; F-:404 |
| **345** | 6-F | 6-$(CH_2)_3$Pim | | 4 | N1:1.7-1.8(2H, m), 2.15(3H, s), 2.2-2.4(8H, m), 2.67 (2H, t), 10.68(1H, s), 14.23(1H, s) ; F-:417 |
| **346** | 5-Me-6-$CO_2$Et | 6-$(CH_2)_3$Morp | | 4 | N1:1.32(3H, t), 2.61(3H, s), 4.26(2H, q), 10.63(1H, s), 14.61(1H, s) ; F+:474 |
| **347** | 6-CH=NOMe | 6-$(CH_2)_3$Morp | | 4 | N1:3.87(3H, s), 8.18(1H, s), 10.70(1H, s), 14.46(1H, s) ; F+:445 |
| **348** | 5-Me-6-$CO_2$Et | 6-$(CH_2)_3$Pim | | 4 | N1:1.32(3H, t), 2.15(3H, s), 2.61(3H, s), 4.26(2H, q), 10.62(1H, s), 14.62(1H, s) ; F+:487 |
| **349** | 6-CH=NOMe | 6-$(CH_2)_3$Pim | | 4 | N1:2.15(3H, s), 3.87(3H, s), 8.18(1H, s), 10.70(1H, s), 14.46(1H, s) ; F+:458 |
| **350** | 6-F | 6-$CH_2$Pim | | 5 | N1: 2.15(3H, s), 2.2-2.6(8H, m), 3.32(2H, s), 10.71(1H, s), 14.24(1H, s) ; F+:391 |
| **351** | 5-Me-6-$CO_2$Et | 6-$CH_2$Pim | | 5 | N1:1.33(3H, t), 2.62(3H, s), 2.87(3H, s), 4.26(2H, s), 4.29(2H, q), 10.70(1H, s), 14.55(1H, s) ; F+:459 |
| **352** | 6-CH=NOMe | 6-$CH_2$Pim | | 5 | N1:3.87(3H, s), 8.18(1H, s), 10.72(1H, s), 14.46(1H, s) ; F+:429 |
| **353** | 5-Me-6-$CO_2$Et | 6-COPim | | 8 | N1:1.32(3H, t), 2.24(3H, s), 2.62(3H, s), 4.26(2H, q), 10.70(1H, s), 14.56(1H, s) ; F+:473 |

Table 20

| Ex | Str | Sy | Dat | Ex | Str | Sy | Dat |
|---|---|---|---|---|---|---|---|
| 30 | 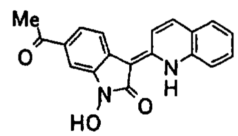 | - | F+:319 | 354 | 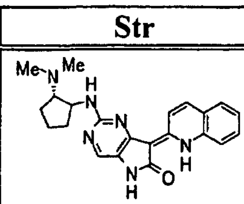 | 18 | F+:389 |
| 355 | 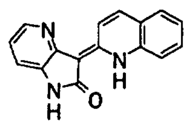 | 17 | F+:262 | 356 | 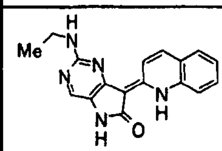 | 18 | F+:306 |
| 357 | 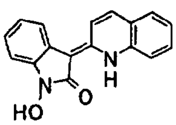 | 30 | F+:277 | 358 | 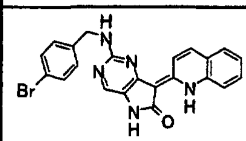 | 18 | F+:446, 448 |
| 359 | 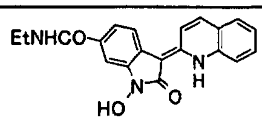 | 30 | F+:348 | 360 | 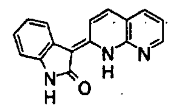 | 19 | F+:262 |
| 361 | 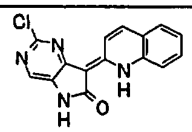 | 17 | F+:297 | 362 | 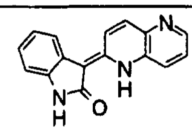 | 19 | F+262 |
| 363 | 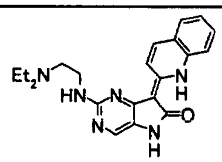 | 18 | F+:377 | 364 | 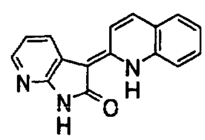 | 19 | F+:262 |
| 365 | 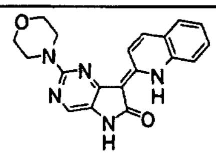 | 18 | F+:348 | 374 | 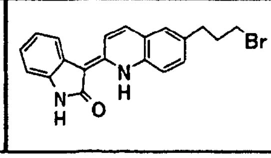 | 19 | F+:380, 382 |
| 375 | 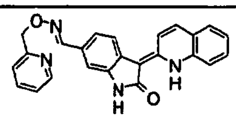 | 29 | N1:5.22(2H,s),8.35(1H,s),10.70(1H,s),14.44(1H,s) ; F+:395 | | | | |
| 376 | 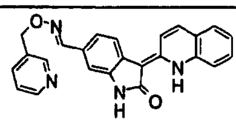 | 29 | N1:5.19(2H,s),8.28(1H,s),10.71(1H,s),14.45(1H,s) ; F+:395 | | | | |
| 377 | 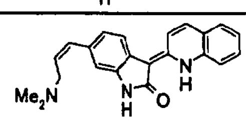 | 17 | N1:2.18(6H,s),3.18(2H,d),5.62(1H,dt),6.52(1H,d),10.60(1H,s), 14.30(1H,s) ; F+:344 | | | | |
| 378 | 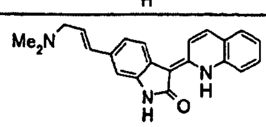 | 17 | N1:2.23(6H,s),3.09(2H,d),6.13(1H,dt),6.54(1H,d),10.60(1H,s),14.28(1H,s) ; F+:344 | | | | |
| 379 | 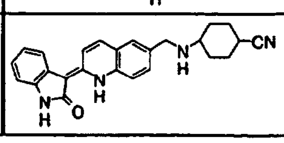 | 5 | N1:2.43-2.50(1H,m),2.98(1H,qui),3.79(2H,s),10.56(1H,s),14.38(1H,s) ; F+:397 | | | | |

**Table 21**

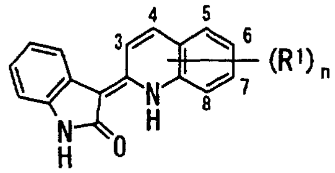

(I)

| Co | $(R^1)n$ | Co | $(R^1)n$ | Co | $(R^1)n$ |
|---|---|---|---|---|---|
| 1 | 5-Me-6-O$(CH_2)_3CO_2H$ | 16 | 4-CONH$(CH_2)_2$OMe-6-$CH_2$-Pim | 31 | 5- NHCO-Pim |
| 2 | 5-$NHSO_2(CH_2)_2$-Morp | 17 | 5-CN-6-O$(CH_2)_3CO_2H$ | 32 | 5-$CH_2$-Pim |
| 3 | 5- $NHCOCH_2$-Morp | 18 | 5-CH=NOMe-6-O$(CH_2)_3CO_2H$ | 33 | 5-$(CH_2)_3$-Pim |
| 4 | 5-NH$(CH_2)_2$OMe | 19 | 6-CH=$CHCO_2H$ | 34 | 5-CO-Pim |
| 5 | 5-C≡C-$CH_2NEt_2$ | 20 | 5-$CH_2$OH-6-O$(CH_2)_3CO_2H$ | 35 | 6-$(CH_2)_2$-Pim |
| 6 | 5-$CH_2CH_2CO_2H$ | 21 | 6-$NHSO_2(CH_2)_2$-Morp | 36 | 6-NHCO-Pim |
| 7 | 4-Cl-6-O$(CH_2)_3CO_2H$ | 22 | 6-NH$(CH_2)_2$OMe | 37 | 6-$SO_2$-Pim |
| 8 | 6-O$(CH_2)_2NHSO_2$Me | 23 | 6-CH=$CHCH_2NEt_2$ | 38 | 6-C≡C-$CO_2H$ |
| 9 | 6-N(Me)CO-Pim | 24 | 5-$CONMe_2$-6-O$(CH_2)_3CO_2H$ | 39 | 6-C≡C-$CH_2NEt_2$ |
| 10 | 6-$CH_2$(4-$CH_2CO_2H$-Pip1) | 25 | 6-CON$(CH_2CO_2H)_2$ | 40 | 6-(4-$CO_2H$-Pip1) |
| 11 | 6-$CH_2$(4-iPr-Pipera) | 26 | 6-CO(4-$(CH_2)_2CO_2H$-Pip1) | 41 | 6-$CH_2$(4-CN-Pip1) |
| 12 | 6-$CH_2$(4-iBu-Pipera) | 27 | 6-$CH_2$(4-$CH_2C(CH_3)_3$-Pipera) | 42 | 6-$(CH_2)_3$-Tet |
| 13 | 6-$CH_2NHCH_2$(4-Me-Py3) | 28 | 6-$CH_2NHCH_2$(4-CN-cHex) | 43 | 6-N$(CH_2CO_2H)_2$ |
| 14 | 6 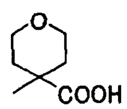 | 29 | 6 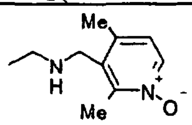 | 44 | 6 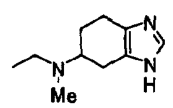 |
| 15 | 6 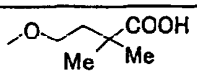 | 30 | 6-O$(CH_2)_2$(4-$(CH_2)_2CO_2H$-Pip1) | | |

**Table 22**

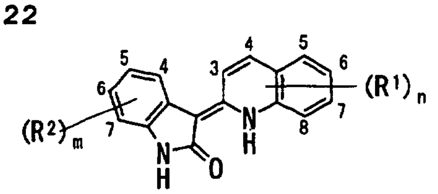

(I)

| Co | $(R^2)m$ | $(R^1)n$ | Co | $(R^2)m$ | $(R^1)n$ |
|---|---|---|---|---|---|
| 45 | 6-F | 5-$CH_2$OH-6-O$(CH_2)_3CO_2H$ | 51 | 6-CH=N-$OCH_2$-Tet | - (n=0) |
| 46 | 6-CH=N-OMe | 5-$CH_2$OH-6-O$(CH_2)_3CO_2H$ | 52 | 6-CH=N-$OCH_2$-Im2 | - (n=0) |
| 47 | 5-Me-6-$CO_2$Et | 6-$(CH_2)_2CO_2H$ | 53 | 6-CH=N-$OCH_2$(6-$NMe_2$-Py2) | - (n=0) |
| 48 | 5-Me-6-$CO_2$Et | 6-O$(CH_2)_3CO_2H$ | 54 | 6-CH=N-$OCH_2$(6-Pim-Py2) | - (n=0) |
| 49 | 6-CH=N-OMe | 6-$(CH_2)_2CO_2H$ | 55 | 6-CH=N-$OCH_2CO_2H$ | - (n=0) |
| 50 | 6-CH=N-OMe | 6-O$(CH_2)_3CO_2H$ | | | |

**Claims**

**1.** A 3-quinolin-2(1H)-ylideneindolin-2-one derivative represented by the following general formula (I) or a salt thereof,

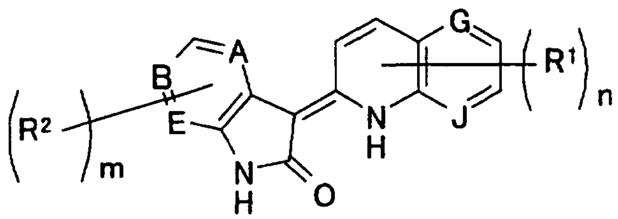

(I)

(symbols in the formula have the following meanings;

A, B, E, G and J: the same or different from one another and each represents N atom or C atom,

$R^1$ and $R^2$: the same or different from each other and each represents a lower alkyl, a lower alkenyl, a lower alkynyl, $R^a$, X-($C_{1-8}$ alkylene which may be substituted by $OR^b$)-$R^a$, X-($C_{1-8}$ alkenylene)-$R^a$ or X-($C_{1-8}$ alkynylene)-$R^a$, with the proviso that each of $R^1$ and $R^2$ does not substitute to the ring nitrogen atom,

X: O, CO, COO, OCO, S, SO, $SO_2$, $NR^b$, $NR^bSO_2$, $SO_2NR^b$, $CONR^b$, $NR^bCO$, $NR^bCONR^c$, $NR^bCOO$, $OCONR^b$ or a bond,

$R^a$: a halogeno lower alkyl, a halogen, $NO_2$, CN, $OR^b$, O-(lower alkylene)-$NR^bR^c$, $COOR^b$, $COR^b$, $CONR^bR^c$, $NR^bR^c$, $NR^d$-(lower alkylene)-$NR^bR^c$, $NR^d$-(lower alkylene)-$OR^b$, N(lower alkylene-$NR^bR^c$)$_2$, $NR^cCOR^b$, $NR^d$-$CONR^bR^c$, $SR^b$, SO-lower alkyl, $SO_2$-lower alkyl, $SO_2$RIN, $SO_2$-(lower alkylene)-RIN, RIN, $SO_2NR^bR^c$, $NR^cSO_2R^b$, $NR^cCOOR^b$, OCO-$NR^bR^c$, OCO-$R^b$, $NR^d$-(lower alkylene)-$COOR^b$, N(lower alkylene-$COOR^b$)$_2$, $CONR^b$-$OR^c$, $CONR^d$-(lower alkylene)-$COOR^b$, CON(lower alkylene-$COOR^b$)$_2$, $CR^d$=N-O-$R^c$, $CR^d$=N-O-(lower alkylene)-CO-$OR^b$ or $CR^d$=N-O-(lower alkylene) -$NR^bR^c$,

$R^b$, $R^c$ and $R^d$: the same or different from one another and each represents H, a lower alkyl, a lower alkylene-RIN or RIN,

RIN: a saturated heterocyclic ring which may have one or more substituents, a cycloalkyl which may have one or more substituents, an aryl which may have one or more substituents or a heteroaryl which may have one or more substituents, and

n and m: the same or different from each other and each is 0 or an integer of from 1 to 4, with the proviso that they are not 0 at the same time when all of A, B, E, G and J are C atom).

**2.** The 3-quinolin-2(1H)-ylideneindolin-2-one derivative or a salt thereof according to claim 1, wherein all of A, B, E, G and J are C atom.

**3.** The 3-quinolin-2(1H)-ylideneindolin-2-one derivative or a salt thereof according to claim 2, wherein m is 0, 1 or 2; $R^2$ is a lower alkyl, $R^{a2}$ or $X^2$-($C_{1-8}$ alkylene)-$R^{a2}$; $X^2$ is O, CO, COO, $NR^b$, $CONR^b$ or a bond; and $R^{a2}$ is a halogeno lower alkyl, a halogen, $NO_2$, CN, $OR^b$, $COOR^b$, $COR^b$, $CONR^bR^c$, $NR^cCOR^b$, $NR^bR^c$, $SO_2$-lower alkyl, RIN, $SO_2NR^bR^c$, OCO-$R^b$, $CONR^b$-$OR^c$, $CR^d$=N-$OR^c$ or $CR^d$=N-O-(lower alkylene) -$NR^bR^c$.

**4.** The 3-quinolin-2(1H)-ylideneindolin-2-one derivative or a salt thereof according to claim 3, wherein n is 0, 1 or 2; $R^1$ is a lower alkyl, $R^{a1}$ or $X^1$-($C_{1-8}$ alkylene which may be substituted by $OR^b$)-$R^{a1}$; $X^1$ is O, $CONR^b$, $NR^bCO$, $NR^bCONR^c$ or a bond; and $R^{a1}$ is a halogen, $NO_2$, CN, $OR^b$, $COOR^b$, $COR^b$, $CONR^bR^c$, $NR^bR^c$, $NR^d$-(lower alkylene)-$NR^bR^c$, $NR^d$-(lower alkylene)-$OR^b$, $NR^dCONR^bR^c$, RIN, OCO-$R^b$, $NR^d$-(lower alkylene)-$COOR^b$ or $CONR^b$-$OR^c$.

**5.** A pharmaceutical composition which comprise a 3-quinolin-2(1H)-ylideneindolin-2-one derivative represented by the following general formula (I') or a salt thereof and a pharmaceutically acceptable carrier,

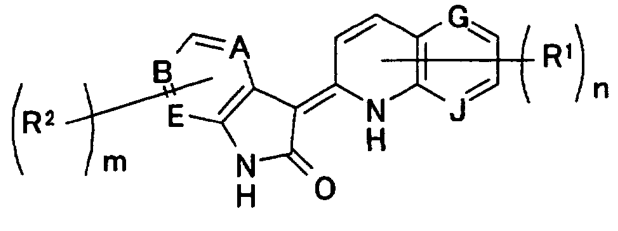

(I')

(symbols in the formula have the following meanings;
A, B, E, G and J: the same or different from one another and each represents N atom or C atom,
$R^1$ and $R^2$: the same or different from each other and each represents a lower alkyl, a lower alkenyl, a lower alkynyl, $R^a$, X-($C_{1-8}$ alkylene which may be substituted by $OR^b$)-$R^a$, X-($C_{1-8}$ alkenylene)-$R^a$ or X-($C_{1-8}$ alkynylene)-$R^a$, with the proviso that each of $R^1$ and $R^2$ does not substitute to the ring nitrogen atom,

X: O, CO, COO, OCO, S, SO, $SO_2$, $NR^b$, $NR^bSO_2$, $SO_2NR^b$, $CONR^b$, $NR^bCO$, $NR^bCONR^c$, $NR^bCOO$, $OCONR^b$ or a bond,

$R^a$: a halogeno lower alkyl, a halogen, $NO_2$, CN, $OR^b$, O-(lower alkylene)-$NR^bR^c$, $COOR^b$, $COR^b$, $CONR^bR^c$, $NR^bR^c$, $NR^d$-(lower alkylene)-$NR^bR^c$, $NR^d$-(lower alkylene)-$OR^b$, N (lower alkylene-$NR^bR^c$)$_2$, $NR^cCOR^b$, $NR^d$-$CONR^bR^c$, $SR^b$, SO-lower alkyl, $SO_2$-lower alkyl, $SO_2$RIN, $SO_2$-(lower alkylene)-RIN, RIN, $SO_2NR^bR^c$, $NR^cSO_2R^b$, $NR^cCOOR^b$, OCO-$NR^bR^c$, OCO-$R^b$, $NR^d$- (lower alkylene)-$COOR^b$, N(lower alkylene-$COOR^b$)$_2$, $CONR^b$-$OR^c$, $CONR^d$-(lower alkylene)-$COOR^b$, CON(lower alkylene-$COOR^b$)$_2$, $CR^d$=N-O-$R^c$, $CR^d$=N-O-(lower alkylene)-CO-$OR^b$ or $CR^d$=N-O-(lower alkylene) -$NR^bR^c$,

$R^b$, $R^c$ and $R^d$: the same or different from one another and each represents H, a lower alkyl, a lower alkylene-RIN or RIN,

RIN: a saturated heterocyclic ring which may have one or more substituents, a cycloalkyl which may have one or more substituents, an aryl which may have one or more substituents or a heteroaryl which may have one or more substituents, and

n and m: the same or different from each other and each is 0 or an integer of from 1 to 4).

**6.** The pharmaceutical composition according to claim 5, which is a vascular endothelial growth factor inhibitor.

**7.** The pharmaceutical composition according to claim 5, which is an angiogenesis inhibitor.

**8.** The pharmaceutical composition according to claim 5, which is an anti-tumor agent.

## INTERNATIONAL SEARCH REPORT

International application No.
PCT/JP02/05014

A. CLASSIFICATION OF SUBJECT MATTER

Int.Cl[7] C07D401/04, 14, 405/14, 409/14, 413/14, 417/14, 471/04, 487/04, 491/113, A61K31/4709, 31/496, 31/501, 31/506, A61K31/5355, 5377, 551, A61P27/02, 35/00, 43/00

According to International Patent Classification (IPC) or to both national classification and IPC

B. FIELDS SEARCHED

Minimum documentation searched (classification system followed by classification symbols)

Int.Cl[7] C07D401/04, 14, 405/14, 409/14, 413/14, 417/14, 471/04, 487/04, 491/113, A61K31/4709, 31/496, 31/501, 31/506, A61K31/5355, 5377, 551, A61P27/02, 35/00, 43/00

Documentation searched other than minimum documentation to the extent that such documents are included in the fields searched

Electronic data base consulted during the international search (name of data base and, where practicable, search terms used)

CAPLUS, REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 00/08202 A1 (Sugen Inc.), 17 February, 2000 (17.02.00), & AU 9954684 A | 1-8 |
| A | WO 99/61422 A (Sugen Inc.), 02 December, 1999 (02.12.99), & AU 9944102 A & EP 1082305 A1 & NO 200005916 A & BR 9910792 A & CZ 200004412 A & SK 200001806 A & CN 1311775 A & KR 2001083039 A & HU 200103617 A & JP 2002-516310 A & US 6395734 B | 1-8 |
| A | WO 99/48868 A2 (Sugen Inc.), 30 September, 1999 (30.09.99), & AU 9933635 A & EP 1066257 A2 & US 6316635 B & JP 2002-507598 A & US 20020028840 A | 1-8 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 July, 2002 (25.07.02) | 06 August, 2002 (06.08.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/05014

C (Continuation). DOCUMENTS CONSIDERED TO BE RELEVANT

| Category* | Citation of document, with indication, where appropriate, of the relevant passages | Relevant to claim No. |
|---|---|---|
| A | WO 99/15500 A1 (Glaxo Group, Ltd.), 01 April, 1999 (01.04.99), & AU 9897407 A & ZA 9808078 A & EP 1009738 A1 & CZ 200000798 A & BR 9812048 A & CN 1278794 A & HU 200004490 A & KR 2001023695 A & JP 2001517652 A & US 6369086 B & US 6387919 B | 1-8 |
| A | WO 99/10349 A1 (Zeneca, Ltd.), 04 March, 1999 (04.03.99), & AU 9888162 A & EP 1005470 A1 & US 6294532 B & JP 2001-514182 A | 1-8 |
| A | WO 97/42187 A1 (Zeneca, Ltd.), 13 November, 1997 (13.11.97), & ZA 9703844 A & AU 9726475 A & EP 912557 A1 & JP 2000-510115 A & US 6265411 B | 1-8 |
| A | Chem. Pharm. Bull., (1970), 18(9), pages 1822-30 | 1 |
| A | Chem. Pharm. Bull., (1971), 19(8), pages 1669-80 | 1 |
| A | Chemical Abstracts, Vol.92, abs. No.76408 | 1 |

Form PCT/ISA/210 (continuation of second sheet) (July 1998)